# EUROPEAN PATENT APPLICATION

(11) **EP 4 733 400 A1**
(43) Date of publication of application: **29.04.2026**
(21) Application number: 24832010.3
(22) Date of filing: 26.06.2024
(51) Int. Cl.: C12N 15/113, A61K 31/7088, A61P 9/00, A61P 19/02, A61P 29/00, A61P 35/00, A61P 43/00

(54) **ANTISENSE OLIGONUCLEOTIDE TARGETING HIC-5**

(30) Priority: 26.06.2023 JP 2023104574
(71) Applicant: SHOWA Medical University, Tokyo 142-8555 (JP); Institute of Science Tokyo, Tokyo 152-8550 (JP)
(72) Inventor: KANEYAMA Shuri, Tokyo 142-8555 (JP); LEI Xiao-Feng, Tokyo 142-8555 (JP); SEIO Kohji, Tokyo 152-8550 (JP); MASAKI Yoshiaki, Tokyo 152-8550 (JP)
(74) Representative: J A Kemp LLP
(86) International application number: PCT/JP2024/023240
(87) International publication number: WO 2025/005151

(57) **Abstract**

The present invention relates to an antisense oligonucleotide targeting a base sequence selected from SEQ ID NOs: 157-312.

## Description

### Technical Field

The present disclosure relates to antisense oligonucleotides targeting Hic-5, methods for treating diseases using the same, etc.

### Background Art

Hydrogen peroxide-inducible clone-5 (Hic-5) is a cell focal adhesion constituent molecule that belongs to the paxillin family. Cell focal adhesions are cellular machineries which connect extracellular matrix (ECM) and intracellular actin skeleton and are constituted by integrin and a plurality of focal adhesion proteins including Hic-5. The cell focal adhesions play an important role in cell adhesion, cell motility, morphological change of cells, and stimulus transmission to cells from surrounding environments, etc. Research using Hic-5 knockout mice, etc., has suggested the possibility that Hic-5 is involved in various diseases (Non-Patent Literatures 1-7). Attempts have also been made to treat various diseases by inhibiting Hic-5 (Patent Literature 1).

### Citation List

### Patent Literatures

Patent Literature 1 WO2021/149329

### Non-Patent Literatures

Non-Patent Literature 1 Kim-Kaneyama et al., J Atheroscler Thromb. 2012;19(7):601-7
Non-Patent Literature 2 Omoto et al., Oncogene. 2018;37(9):1205-1219
Non-Patent Literature 3 Petropoulos et al., J Cell Biol. 2016;213(5):585-99
Non-Patent Literature 4 Lei et al., J Hepatol. 2016;64(1):110-7
Non-Patent Literature 5 Jamba et al., PLoS One. 2015;10(4):e0122773
Non-Patent Literature 6 Arita-Okubo et al., Cardiovasc Res. 2015;105(3):361-71
Non-Patent Literature 7 Yund et al., J Mol Cell Cardiol. 2009;47(4):520-7

### Summary of the Invention

### Technical Problem

Further therapeutic drugs targeting Hic-5 are needed.

### Solution to Problem

Some aspects of the present disclosure relate to the following.
[1] An antisense oligonucleotide targeting the base sequence selected from SEQ ID NOs:157 to 312.
[2] The antisense oligonucleotide according to [1], comprising the base sequence selected from SEQ ID NOs:1 to 156.
[3] The antisense oligonucleotide according to [1] or [2], having modifications on the nucleotides at the 5' terminal portion and 3' terminal portion.
[4] The antisense oligonucleotide according to any one of [1] to [3], consisting of the sequence shown in SEQ ID NOs:313 to 485.
[5] A composition comprising the antisense oligonucleotide according to any one of [1] to [4].
[6] The antisense oligonucleotide according to any one of [1] to [4] or the composition according to [5] for use in treating a disease associated with Hic-5.
[7] The antisense oligonucleotide or composition for use according to [6], wherein the disease associated with Hic-5 is selected from vascular diseases, fibrotic diseases, cardiac hypertrophy, osteoarthritis, and tumors.
[8] A composition for use in treating sclerosing cholangitis, comprising a Hic-5 inhibitor.

### Effect of the Invention

The present disclosure provides antisense oligonucleotides against Hic-5 with excellent properties.

### Brief Description of Drawings

[FIG. 1] FIG. 1 is a dose-response curve of each ASO in KMST-6 cells.
[FIG. 2] FIG. 2 is a dose-response curve of each ASO in JTC-19 cells.
[FIG. 3] FIG. 3 is a dose-response curve of each ASO in human HSC.
[FIG. 4] FIG. 4 is a graph showing Hic-5 mRNA expression levels (left) and Hic-5 mRNA retention rates (right) of each group (mean±SEM). **P<0.01 (Dunnett's multiple comparison test after one-way ANOVA)
[FIG. 5] FIG. 5 is a graph showing Hic-5 protein expression levels of each group (mean±SEM). *P<0.05, **P<0.01 (Dunnett's multiple comparison test after one-way ANOVA)
[FIG. 6] FIG. 6 is a graph showing the results of biochemical tests (mean±SEM).
[FIG. 7] FIG. 7 is a photographic representation showing typical liver tissue images of each experimental group.
[FIG. 8] FIG. 8 is a graph showing fibrotic area (MT-positive area) of each group (mean±SEM). *P<0.05, **P<0.01 (Dunnett's multiple comparison test after one-way ANOVA)
[FIG. 9] FIG. 9 is a graph showing liver weight to body weight ratio (A), plasma AST (B), plasma ALT (C), plasma total bilirubin (D), plasma direct bilirubin (E), and Hic-5 mRNA expression levels (F) of each group (mean±SEM). *P<0.05, **P<0.01 (unpaired two-tailed t-test).
[FIG. 10] FIG. 10 is a photographic representation showing typical HE staining, SR staining, ORO staining (Oil Red), and Hic-5 immunostaining images of liver tissue from each group. The scale bar indicates 200 µm.
[FIG. 11] FIG. 11 is a graph showing the degree of liver fibrosis (Fibrosis), the degree of steatosis (Steatosis), and the mRNA expression levels of Hic-5, Col1a1, Col1a2, Lox, Loxl1, and Loxl2 (mean±SEM). *P<0.05, **P<0.01 (Dunnett's multiple comparison test after one-way ANOVA)
[FIG. 12] FIG. 12 is a graph showing the results of OARSI score.
[FIG. 13] FIG. 13 is a photographic representation showing typical Safranin-O staining images of each group.

### Description of Embodiments

Unless otherwise defined herein, all technical and scientific terms used herein have the same meaning as commonly understood by one of ordinary skill in the art. All patents, applications, and other publications (including online information) referred to herein are incorporated by reference in their entirety. This specification also includes the contents described in the specification and drawings of Japanese Patent Application No. 2023-104574, filed on June 26, 2023, which forms the basis of the priority claim of this application.

In one aspect, the present disclosure relates to antisense oligonucleotides (ASO) targeting Hic-5 (hereinafter, may be referred to as "ASO of the present disclosure").

As is well known in the art, ASOs are oligonucleotides that specifically hybridize to RNA molecules (e.g., mRNA (mature mRNA), pre-mRNA, ncRNA, miRNA, etc., related to the target gene) and exert an influence on the function of the target gene. The influence on the function of the target gene includes, for example, suppression of target gene expression by cleavage of RNA molecules via RNase H.

The ASO of the present disclosure targets specific base sequences of Hic-5 mRNA or pre-mRNA. Therefore, the ASO of the present disclosure can hybridize to its base sequence and exert an influence on the function of the Hic-5 gene. The ASO of the present disclosure typically includes a base sequence complementary to its base sequence. The degree of complementarity may be, for example, 90% or more, 91% or more, 92% or more, 93% or more, 94% or more, 95% or more, 96% or more, 97% or more, 98% or more, 99% or more, or 100% relative to the full length of the target base sequence.

In some embodiments, the ASO of the present disclosure targets a base sequence selected from SEQ ID NOs:157 to 312. In one embodiment, the ASO of the present disclosure targets a base sequence related to mouse Hic-5 selected from SEQ ID NOs:157 to 224. In another embodiment, the ASO of the present disclosure targets a base sequence related to human Hic-5 selected from SEQ ID NOs:163 to 171 and 225 to 312. In a preferred embodiment, the ASO of the present disclosure targets a base sequence selected from SEQ ID NOs:163 to 165, 237, and 250.

In some preferred embodiments, the ASO of the present disclosure targets the target sequence of the ASO selected from ASO Nos:H10 to H29, H31, H35, H36, H38, H39, H41, H42, and H44 to H53 (any one of SEQ ID NOs:234 to 253, 255, 259, 260, 262, 263, 265, 266, and 268 to 277). In another preferred embodiment, the ASO of the present disclosure targets the target sequence of the ASO selected from ASO numbers H11 to H15, H19, H25 to H31, H36, H38, and H44 to H53 (any one of SEQ ID NOs:235 to 239, 243, 249 to 255, 260, 262, and 268 to 277). In another preferred embodiment, the ASO of the present disclosure targets the target sequence of the ASO selectedfrom ASO numbers H8, H13, H16 to H18, H21, and H23 to H28 (any one of SEQ ID NOs:232, 237, 240 to 242, 245, and 247 to 252). In another preferred embodiment, the ASO of the present disclosure targets the target sequence of the ASO selected from ASO numbers M1 to M10, M12 to M20, M22, M23, M25, M26, M29, M30, M33 to M48, M50 to M56, and M67 (any one of SEQ ID NOs:157 to 166, 168 to 176, 178, 179, 181, 182, 185, 186, 189 to 204, 206 to 212, and 223). In another preferred embodiment, the ASO of the present disclosure targets the target sequence of the ASO selected from ASO numbers M2 to M5, M7 to M16, M23, M28, M29, M33 to M35, M46, and M48 to M50 (any one of SEQ ID NOs:158 to 161, 163 to 172, 179, 184, 185, 189 to 191, 202, and 204 to 206). In another preferred embodiment, the ASO of the present disclosure targets the target sequence of the ASO selected from ASO numbers H1 to H3, H8, H10 to H18, H20 to H29, H36, H38 to H42, and H44 to H53 (any one of SEQ ID NOs:225 to 227, 232, 234 to 242, 244 to 253, 260, 262 to 266, and 268 to 277). In another preferred embodiment, the ASO of the present disclosure targets the target sequence of the ASO selected from ASO numbers H1687, H1790, H1790_18, H1791, H1791_18, H1994_18, H1995_18, H1996_18, H2002_18, H2003_18, H2004_18, H2005_18, H2007_18, H2008_18, H2010_18, H2576_18, H2577, H2577_17, H2578_18, H26, H26_1, H26_2, H26_3, H26A4_1, H26A4_2, H26A4_3, H26A5_1, H26A5_2, H26A5_3, H3974_1, H3974_2, H3975, H3977_18, H3978_17, H3979_18, H3980_17, H4745_18, H4801_17, and H4802_18 (any one of SEQ ID NOs:250, 278 to 299, 301, 305 to 309, 311, and 312). In another preferred embodiment, the ASO of the present disclosure targets the target sequence of the ASO selected from ASO numbers H1687, H2577_17, H26, H26 cho, H26 toco, H26_1, H26_2, H26_3, H26A4_1, H26A4_2, H26A4_3, H26A5_1, H26A5_2, H26A5_3, H3979_18, H3980_17, H4745_18, H4801_17, and H4802_18 (any one of SEQ ID NOs:250, 278, 294, 297, 298, 307 to 309, 311, and 312). In another preferred embodiment, the ASO of the present disclosure targets the target sequence of the ASO selected from ASO numbers H26, H3979_18_43_2, H3979_18_43_3, and H4745_18 (any one of SEQ ID NOs:250, 307, and 309). In another preferred embodiment, the ASO of the present disclosure targets the target sequence of the ASO selected from ASO numbers H1687, H1687_16_32_1, H1687_16_32_2, H26, H26A4_3, H26A5_3_, H3979_18, H3979_18_33_1, H3979_18_33_2, H3979_18_43_1, H3979_18_43_2, H3979_18_43_3, and H4745_18 (any one of SEQ ID NOs:250, 278, 297, 298, 307, and 309). In another preferred embodiment, the ASO of the present disclosure targets the target sequence of the ASO selected from ASO numbers H26, H26A4_3, H26A5_3, H3979_18, H3979_18_33_1, H3979_18_43_1, H3979_18_43_2, H3979_18_43_3, and H4745_18 (any one of SEQ ID NOs:250, 297, 298, 307, and 309).

In some embodiments, the ASO of the present disclosure comprises or consists of the base sequence selected from SEQ ID NOs:1 to 156. In one embodiment, the ASO of the present disclosure comprises or consists of the base sequence selected from SEQ ID NOs:1 to 68. In another embodiment, the ASO of the present disclosure comprises or consists of the base sequence selected from SEQ ID NOs:7 to 15 and 67 to 156. In a preferred embodiment, the ASO of the present disclosure comprises or consists of the base sequence selected from SEQ ID NOs:7 to 9, 81, and 94. The ASO of the present disclosure may also comprise or consist of the base sequence having a sequence identity of 90% or more, 91% or more, 92% or more, 93% or more, 94% or more, 95% or more, 96% or more, 97% or more, 98% or more, or 99% or more relative to the full length of the above base sequence.

In some preferred embodiments, the ASO of the present disclosure comprises the base sequence of the ASO selected from ASO numbers H10 to H29, H31, H35, H36, H38, H39, H41, H42, and H44 to H53 (any one of SEQ ID NOs:78 to 97, 99, 103, 104, 106, 107, 109, 110, and 112 to 121). In another preferred embodiment, the ASO of the present disclosure comprises the base sequence of the ASO selected from ASO numbers H11 to H15, H19, H25 to H31, H36, H38, and H44 to H53 (any one of SEQ ID NOs:79 to 83, 87, 93 to 99, 104, 106, and 112 to 121). In another preferred embodiment, the ASO of the present disclosure comprises the base sequence of the ASO selected from ASO numbers H8, H13, H16 to H18, H21, and H23 to H28 (any one of SEQ ID NOs:76, 81, 84 to 86, 89, and 91 to 96). In another preferred embodiment, the ASO of the present disclosure comprises the base sequence of the ASO selected from ASO numbers M1 to M10, M12 to M20, M22, M23, M25, M26, M29, M30, M33 to M48, M50 to M56, and M67 (SEQ ID NOs:1 to 10, 12 to 20, 22, 23, 25, 26, 29, 30, 33 to 48, 50 to 56, and 67). In another preferred embodiment, the ASO of the present disclosure comprises the base sequence of the ASO selected from ASO numbers M2 to M5, M7 to M16, M23, M28, M29, M33 to M35, M46, and M48 to M50 (SEQ ID NOs:2 to 5, 7 to 16, 23, 28, 29, 33 to 35, 46, and 48 to 50). In another preferred embodiment, the ASO of the present disclosure comprises the base sequence of the ASO selected from ASO numbers H1 to H3, H8, H10 to H18, H20 to H29, H36, H38 to H42, and H44 to H53 (any one of SEQ ID NOs:69 to 71, 76, 78 to 86, 88 to 97, 104, 106 to 110, and 112 to 121). In another preferred embodiment, the ASO of the present disclosure comprises the base sequence of the ASO selected from ASO numbers H1687, H1790, H1790_18, H1791, H1791_18, H1994_18, H1995_18, H1996_18, H2002_18, H2003_18, H2004_18, H2005_18, H2007_18, H2008_18, H2010_18, H2576_18, H2577, H2577_17, H2578_18, H26, H26_1, H26_2, H26_3, H26A4_1, H26A4_2, H26A4_3, H26A5_1, H26A5_2, H26A5_3, H3974_1, H3974_2, H3975, H3977_18, H3978_17, H3979_18, H3980_17, H4745_18, H4801_17, and H4802_18 (any one of SEQ ID NOs:94, 122 to 143, 145, 149 to 153, 155, and 156). In another preferred embodiment, the ASO of the present disclosure comprises the base sequence of the ASO selected from ASO numbers H1687, H2577_17, H26, H26 cho, H26 toco, H26_1, H26_2, H26_3, H26A4_1, H26A4_2, H26A4_3, H26A5_1, H26A5_2, H26A5_3, H3979_18, H3980_17, H4745_18, H4801_17, and H4802_18 (any one of SEQ ID NOs:94, 122, 138, 141, 142, 151 to 153, 155, and 156). In another preferred embodiment, the ASO of the present disclosure comprises the base sequence of the ASO selected from ASO numbers H26, H3979_18_43_2, H3979_18_43_3, and H4745_18 (any one of SEQ ID NOs:94, 151, and 153). In another preferred embodiment, the ASO of the present disclosure comprises the base sequence of the ASO selected from ASO numbers H1687, H1687_16_32_1, H1687_16_32_2, H26, H26A4_3, H26A5_3_, H3979_18, H3979_18_33_1, H3979_18_33_2, H3979_18_43_1, H3979_18_43_2, H3979_18_43_3, and H4745_18 (any one of SEQ ID NOs:94, 122, 141, 142, 151, and 153). In another preferred embodiment, the ASO of the present disclosure comprises the base sequence of the ASO selected from ASO numbers H26, H26A4_3, H26A5_3, H3979_18, H3979_18_33_1, H3979_18_43_1, H3979_18_43_2, H3979_18_43_3, and H4745_18 (any one of SEQ ID NOs:94, 141, 142, 151, and 153).

Table 1 shows the base sequences and target sequences included in the ASO of the present disclosure.

**[Table 1-1]**

| Table 1 Base sequences and target sequences included in the ASO of the present disclosure | | | | |
|---|---|---|---|---|
| ASO Number | Sequence | Sequence Number | Target Sequence | Sequence Number |
| M1 | TGTGTGAAGTGGTGGT | 1 | ACCACCACTTCACACA | 157 |
| M2 | ATGTGTGAAGTGGTGG | 2 | CCACCACTTCACACAT | 158 |
| M3 | CATGTGTGAAGTGGTG | 3 | CACCACTTCACACATG | 159 |
| M4 | TGGCCATAGGGTGG | 4 | CCACCCTATGGCCA | 160 |
| M5 | GTGGCCATAGGGTG | 5 | CACCCTATGGCCAC | 161 |
| M6 | TGTGCTGTATAGATGA | 6 | TCATCTATACAGCACA | 162 |
| M7 | AACTGAGACATGATTT | 7 | AAATCATGTCTCAGTT | 163 |
| M8 | GAACTGAGACATGATT | 8 | AATCATGTCTCAGTTC | 164 |
| M9 | GGAACTGAGACATGAT | 9 | ATCATGTCTCAGTTCC | 165 |
| M10 | GGGAACTGAGACATGA | 10 | TCATGTCTCAGTTCCC | 166 |
| M11 | TGGGAACTGAGACATG | 11 | CATGTCTCAGTTCCCA | 167 |
| M12 | ATGGGAACTGAGACAT | 12 | ATGTCTCAGTTCCCAT | 168 |
| M13 | GATGGGAACTGAGACA | 13 | TGTCTCAGTTCCCATC | 169 |
| M14 | AGATGGGAACTGAGAC | 14 | GTCTCAGTTCCCATCT | 170 |
| M15 | TAGATGGGAACTGAGA | 15 | TCTCAGTTCCCATCTA | 171 |
| M16 | GGGTGAGCTCTTGTCT | 16 | AGACAAGAGCTCACCC | 172 |
| M17 | TGGGTGAGCTCTTGTC | 17 | GACAAGAGCTCACCCA | 173 |
| M18 | GTGGGTGAGCTCTTGT | 18 | ACAAGAGCTCACCCAC | 174 |
| M19 | AGTGGGTGAGCTCTTG | 19 | CAAGAGCTCACCCACT | 175 |
| M20 | CAGTGGGTGAGCTCTT | 20 | AAGAGCTCACCCACTG | 176 |
| M21 | CTGAAGGCTTTGAGGG | 21 | CCCTCAAAGCCTTCAG | 177 |
| M22 | GCTGAAGGCTTTGAGG | 22 | CCTCAAAGCCTTCAGC | 178 |
| M23 | GGCTGAAGGCTTTGAG | 23 | CTCAAAGCCTTCAGCC | 179 |
| M24 | TGGCTGAAGGCTTTGA | 24 | TCAAAGCCTTCAGCCA | 180 |
| M25 | TTCCTGAGTGGCGGAG | 25 | CTCCGCCACTCAGGAA | 181 |
| M26 | TCCTGAGTGGCGGA | 26 | TCCGCCACTCAGGA | 182 |
| M27 | TTCCTGAGTGGCGG | 27 | CCGCCACTCAGGAA | 183 |
| M28 | CAGTTCCTGAGTGGCG | 28 | CGCCACTCAGGAACTG | 184 |
| M29 | CCAGTTCCTGAGTGGC | 29 | GCCACTCAGGAACTGG | 185 |
| M30 | TCCAGTTCCTGAGTGG | 30 | CCACTCAGGAACTGGA | 186 |
| M31 | ATCCAGTTCCTGAGTG | 31 | CACTCAGGAACTGGAT | 187 |
| M32 | TATCCAGTTCCTGAGT | 32 | ACTCAGGAACTGGATA | 188 |
| M33 | TTGCCCAGCTATAGGT | 33 | ACCTATAGCTGGGCAA | 189 |
| M34 | CTTGCCCAGCTATAGG | 34 | CCTATAGCTGGGCAAG | 190 |
| M35 | ACTTGCCCAGCTATAG | 35 | CTATAGCTGGGCAAGT | 191 |
| M36 | TTTTGTGTCGGATGGG | 36 | CCCATCCGACACAAAA | 192 |
| M37 | ATTTTGTGTCGGATGG | 37 | CCATCCGACACAAAAT | 193 |
| M38 | CATTTTGTGTCGGATG | 38 | CATCCGACACAAAATG | 194 |
| M39 | CCATTTTGTGTCGGAT | 39 | ATCCGACACAAAATGG | 195 |
| M40 | ACCATTTTGTGTCGGA | 40 | TCCGACACAAAATGGT | 196 |
| M41 | AGCATGGAAATGGTTT | 41 | AAACCATTTCCATGCT | 197 |
| M42 | GAGCATGGAAATGGTT | 42 | AACCATTTCCATGCTC | 198 |
| M43 | TGAGCATGGAAATGGT | 43 | ACCATTTCCATGCTCA | 199 |
| M44 | CTGAGCATGGAAATGG | 44 | CCATTTCCATGCTCAG | 200 |
| M45 | GCTGAGCATGGAAATG | 45 | CATTTCCATGCTCAGC | 201 |

**[Table 1-2]**

| | | | | |
|---|---|---|---|---|
| M46 | AGCCTTTGGTGAGTGG | 46 | CCACTCACCAAAGGCT | 202 |
| M47 | GAGCCTTTGGTGAGTG | 47 | CACTCACCAAAGGCTC | 203 |
| M48 | GGAGCCTTTGGTGAGT | 48 | ACTCACCAAAGGCTCC | 204 |
| M49 | AGGAGCCTTTGGTGAG | 49 | CTCACCAAAGGCTCCT | 205 |
| M50 | AAGGAGCCTTTGGTGA | 50 | TCACCAAAGGCTCCTT | 206 |
| M51 | TTTTGAAGGCTTAGTT | 51 | AACTAAGCCTTCAAAA | 207 |
| M52 | TTTTTGAAGGCTTAGT | 52 | ACTAAGCCTTCAAAAA | 208 |
| M53 | GTTTTTGAAGGCTTAG | 53 | CTAAGCCTTCAAAAAC | 209 |
| M54 | TGTTTTTGAAGGCTTA | 54 | TAAGCCTTCAAAAACA | 210 |
| M55 | GTGTTTTTGAAGGCTT | 55 | AAGCCTTCAAAAACAC | 211 |
| M56 | GCGGACAGCTTGGG | 56 | CCCAAGCTGTCCGC | 212 |
| M57 | GGCGGACAGCTTGG | 57 | CCAAGCTGTCCGCC | 213 |
| M58 | TGGCGGACAGCTTG | 58 | CAAGCTGTCCGCCA | 214 |
| M59 | CTGGCGGACAGCTT | 59 | AAGCTGTCCGCCAG | 215 |
| M60 | GCTGGCGGACAGCT | 60 | AGCTGTCCGCCAGC | 216 |
| M61 | TGGCGGTGCGCTGG | 61 | CCAGCGCACCGCCA | 217 |
| M62 | ATGGCGGTGCGCTG | 62 | CAGCGCACCGCCAT | 218 |
| M63 | CATGGCGGTGCGCT | 63 | AGCGCACCGCCATG | 219 |
| M64 | CCATGGCGGTGCGC | 64 | GCGCACCGCCATGG | 220 |
| M65 | TCCATGGCGGTGCG | 65 | CGCACCGCCATGGA | 221 |
| M66 | CTCCATGGCGGTGC | 66 | GCACCGCCATGGAG | 222 |
| M67 | GGCCATAGGGTGGG | 67 | CCCACCCTATGGCC | 223 |
| M68 | CTGAGTGGCGGAGG | 68 | CCTCCGCCACTCAG | 224 |
| H1 | AGAGGAGAATGGAGGG | 69 | CCCTCCATTCTCCTCT | 225 |
| H2 | AAGAGGAGAATGGAGG | 70 | CCTCCATTCTCCTCTT | 226 |
| H3 | GAAGAGGAGAATGGAG | 71 | CTCCATTCTCCTCTTC | 227 |
| H4 | GTCTTTTCTTATCTTC | 72 | GAAGATAAGAAAAGAC | 228 |
| H5 | GGTCTTTTCTTATCTT | 73 | AAGATAAGAAAAGACC | 229 |
| H6 | GGGTCTTTTCTTATCT | 74 | AGATAAGAAAAGACCC | 230 |
| H7 | TGGGTCTTTTCTTATC | 75 | GATAAGAAAAGACCCA | 231 |
| H8 | CTGGGTCTTTTCTTAT | 76 | ATAAGAAAAGACCCAG | 232 |
| H9 | GAAGTCAGAGAGTGAG | 77 | CTCACTCTCTGACTTC | 233 |
| H10 | GGAAGTCAGAGAGTGA | 78 | TCACTCTCTGACTTCC | 234 |
| H11 | CGGAAGTCAGAGAGTG | 79 | CACTCTCTGACTTCCG | 235 |
| H12 | GCGGAAGTCAGAGAGT | 80 | ACTCTCTGACTTCCGC | 236 |
| H13 | CGCGGAAGTCAGAGAG | 81 | CTCTCTGACTTCCGCG | 237 |
| H14 | ACGCGGAAGTCAGAGA | 82 | TCTCTGACTTCCGCGT | 238 |
| H15 | AACGCGGAAGTCAGAG | 83 | CTCTGACTTCCGCGTT | 239 |
| H16 | GAACGCGGAAGTCAGA | 84 | TCTGACTTCCGCGTTC | 240 |
| H17 | TGAACGCGGAAGTCAG | 85 | CTGACTTCCGCGTTCA | 241 |
| H18 | TTGAACGCGGAAGTCA | 86 | TGACTTCCGCGTTCAA | 242 |
| H19 | CCCCAGCATGGTGTCT | 87 | AGACACCATGCTGGGG | 243 |
| H20 | TGCCTCCCAGGGCGGT | 88 | ACCGCCCTGGGAGGCA | 244 |
| H21 | ATGGGCTGGTTGCAGA | 89 | TCTGCAACCAGCCCAT | 245 |
| H22 | GATGGGCTGGTTGCAG | 90 | CTGCAACCAGCCCATC | 246 |
| H23 | CGTGGAAACCCTCATC | 91 | GATGAGGGTTTCCACG | 247 |

**[Table 1-3]**

| | | | | |
|---|---|---|---|---|
| H24 | TCGTGGAAACCCTCAT | 92 | ATGAGGGTTTCCACGA | 248 |
| H25 | CTCGTGGAAACCCTCA | 93 | TGAGGGTTTCCACGAG | 249 |
| H26, H26_1, H26_2, H26_3, H26_cho, H26_toco | GCTCGTGGAAACCCTC | 94 | GAGGGTTTCCACGAGC | 250 |
| H27 | TCCTCCATGGCGGG | 95 | CCCGCCATGGAGGA | 251 |
| H28 | GTCCTCCATGGCGG | 96 | CCGCCATGGAGGAC | 252 |
| H29 | GGTCCTCCATGGCG | 97 | CGCCATGGAGGACC | 253 |
| H30 | CGCAGGGCGCTCTT | 98 | AAGAGCGCCCTGCG | 254 |
| H31 | CCGCAGGGCGCTCT | 99 | AGAGCGCCCTGCGG | 255 |
| H32 | TCCGCAGGGCGCTC | 100 | GAGCGCCCTGCGGA | 256 |
| H33 | CTCCGCAGGGCGCT | 101 | AGCGCCCTGCGGAG | 257 |
| H34 | CCCAGCATGGTGTC | 102 | GACACCATGCTGGG | 258 |
| H35 | CCCCAGCATGGTGT | 103 | ACACCATGCTGGGG | 259 |
| H36 | GCCCCAGCATGGTG | 104 | CACCATGCTGGGGC | 260 |
| H37 | AGCCCCAGCATGGT | 105 | ACCATGCTGGGGCT | 261 |
| H38 | CCCAGGGCGGTGGA | 106 | TCCACCGCCCTGGG | 262 |
| H39 | TCCCAGGGCGGTGG | 107 | CCACCGCCCTGGGA | 263 |
| H40 | CTCCCAGGGCGGTG | 108 | CACCGCCCTGGGAG | 264 |
| H41 | ATGGGCTGGTTGCA | 109 | TGCAACCAGCCCAT | 265 |
| H42 | GATGGGCTGGTTGC | 110 | GCAACCAGCCCATC | 266 |
| H43 | CTCGTGGAAACCCT | 111 | AGGGTTTCCACGAG | 267 |
| H44 | GGGTGGAAGCGGCG | 112 | CGCCGCTTCCACCC | 268 |
| H45 | CGGGTGGAAGCGGC | 113 | GCCGCTTCCACCCG | 269 |
| H46 | CCGGGTGGAAGCGG | 114 | CCGCTTCCACCCGG | 270 |
| H47 | TCCGGGTGGAAGCG | 115 | CGCTTCCACCCGGA | 271 |
| H48 | GTCCGGGTGGAAGC | 116 | GCTTCCACCCGGAC | 272 |
| H49 | GCGCAGGCAGAAGG | 117 | CCTTCTGCCTGCGC | 273 |
| H50 | GGCGCAGGCAGAAG | 118 | CTTCTGCCTGCGCC | 274 |
| H51 | GGGCGCAGGCAGAA | 119 | TTCTGCCTGCGCCC | 275 |
| H52 | CGGGCGCAGGCAGA | 120 | TCTGCCTGCGCCCG | 276 |
| H53 | GCGGGCGCAGGCAG | 121 | CTGCCTGCGCCCGC | 277 |
| H1687, H1687_16_32_1, H1687_16_32_2 | ACCGAGGCTTGCATAC | 122 | GTATGCAAGCCTCGGT | 278 |
| H1790 | GGCATTAAGTTCCTGAA | 123 | TTCAGGAACTTAATGCC | 279 |
| H1790_18 | TGGCATTAAGTTCCTGAA | 124 | TTCAGGAACTTAATGCCA | 280 |
| H1791 | TGGCATTAAGTTCCTGA | 125 | TCAGGAACTTAATGCCA | 281 |
| H1791_18 | GTGGCATTAAGTTCCTGA | 126 | TCAGGAACTTAATGCCAC | 282 |
| H1994_18 | ACATGATTTCATCTGGAG | 127 | CTCCAGATGAAATCATGT | 283 |
| H1995_18 | GACATGATTTCATCTGGA | 128 | TCCAGATGAAATCATGTC | 284 |
| H1996_18 | AGACATGATTTCATCTGG | 129 | CCAGATGAAATCATGTCT | 285 |
| H2002_18 | GAACTGAGACATGATTTC | 130 | GAAATCATGTCTCAGTTC | 286 |
| H2003_18 | GGAACTGAGACATGATTT | 131 | AAATCATGTCTCAGTTCC | 287 |
| H2004_18 | GGGAACTGAGACATGATT | 132 | AATCATGTCTCAGTTCCC | 288 |
| H2005_18 | TGGGAACTGAGACATGAT | 133 | ATCATGTCTCAGTTCCCA | 289 |
| H2007_18 | GATGGGAACTGAGACATG | 134 | CATGTCTCAGTTCCCATC | 290 |
| H2008_18 | AGATGGGAACTGAGACAT | 135 | ATGTCTCAGTTCCCATCT | 291 |

**[Table 1-4]**

| | | | | |
|---|---|---|---|---|
| H2010_18 | CTAGATGGGAACTGAGAC | 136 | GTCTCAGTTCCCATCTAG | 292 |
| H2576_18 | AGGTTTATTGCAGGAGCC | 137 | GGCTCCTGCAATAAACCT | 293 |
| H2577 | TAGGTTTATTGCAGGAGC | 138 | GCTCCTGCAATAAACCTA | 294 |
| H2577_17 | AGGTTTATTGCAGGAGC | 139 | GCTCCTGCAATAAACCT | 295 |
| H2578_18 | ATAGGTTTATTGCAGGAG | 140 | CTCCTGCAATAAACCTAT | 296 |
| H26A4_1, H26A4_2, H26A4_3 | CGCTCGTGGAAACCCT | 141 | AGGGTTTCCACGAGCG | 297 |
| H26A5_1, H26A5_2, H26A5_3 | CGCTCGTGGAAACCCTC | 142 | GAGGGTTTCCACGAGCG | 298 |
| H3974_1, H3974_2 | GCGCTCAAAGTAGCACTC | 143 | GAGTGCTACTTTGAGCGC | 299 |
| H3974_17 | CGCTCAAAGTAGCACTC | 144 | GAGTGCTACTTTGAGCG | 300 |
| H3975 | GCGCTCAAAGTAGCACT | 145 | AGTGCTACTTTGAGCGC | 301 |
| H3975_18 | AGCGCTCAAAGTAGCACT | 146 | AGTGCTACTTTGAGCGCT | 302 |
| H3976 | AGCGCTCAAAGTAGCAC | 147 | GTGCTACTTTGAGCGCT | 303 |
| H3976_17 | AGCGCTCAAAGTAGCAC | 148 | GTGCTACTTTGAGCGCT | 304 |
| H3977_18 | GAAGCGCTCAAAGTAGCA | 149 | TGCTACTTTGAGCGCTTC | 305 |
| H3978_17 | GAAGCGCTCAAAGTAGC | 150 | GCTACTTTGAGCGCTTC | 306 |
| H3979_18, H3979_18_33_1, H3979_18_33_2, H3979_18_43_1, H3979_18_43_2, H3979_18_43_3 | GAGAAGCGCTCAAAGTAG | 151 | CTACTTTGAGCGCTTCTC | 307 |
| H3980_17 | GAGAAGCGCTCAAAGTA | 152 | TACTTTGAGCGCTTCTC | 308 |
| H4745_18 | CAGCTGCAGGAAGTCCCG | 153 | CGGGACTTCCTGCAGCTG | 309 |
| H4800_18 | CCGAGATGTAGTTATCCA | 154 | TGGATAACTACATCTCGG | 310 |
| H4801_17 | CCGAGATGTAGTTATCC | 155 | GGATAACTACATCTCGG | 311 |
| H4802_16 | CCGAGATGTAGTTATC | 156 | GATAACTACATCTCGG | 312 |

In some embodiments, the ASO of the present disclosure targets a base sequence contained in one exon of Hic-5 mRNA. In another embodiment, the ASO of the present disclosure targets a base sequence spanning two exons of Hic-5 mRNA. In yet another embodiment, the ASO of the present disclosure targets a base sequence spanning an intron and exon of Hic-5 pre-mRNA.

In some embodiments, the ASO of the present disclosure targets human Hic-5. In another embodiment, the ASO of the present disclosure targets mouse Hic-5. In certain embodiments, the ASO of the present disclosure targets a base sequence common to human Hic-5 and mouse Hic-5. The base sequences of human Hic-5 mRNA (NM_001042454.3) and non-spliced transcript (ENST00000394863.8) are shown as SEQ ID NOs:503 and 504, and the base sequences of mouse Hic-5 mRNA (NM_001289553.1) and non-spliced transcript (ENSMUST00000164710.8) are shown as SEQ ID NOs:505 and 506, respectively.

The length of the ASO of the present disclosure is not particularly limited as long as it can function as an ASO, but may be, for example, in the range of 10 to 50 nucleotides, 11 to 40 nucleotides, 12 to 35 nucleotides, 13 to 30 nucleotides, or 14 to 28 nucleotides. In certain embodiments, the length of the ASO of the present disclosure may be 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides.

The ASO may include unmodified nucleotides and/or modified nucleotides. In the present specification, the unmodified nucleotide and the modified nucleotide are simply referred to as "nucleotide" collectively. The unmodified nucleotide refers to a naturally occurring nucleotide constituting DNA or RNA, i.e., the one constituted by a nucleobase (adenine, guanine, uracil, thymine, or cytosine), a sugar (ribose or deoxyribose), and a phosphate group. In an unmodified nucleic acid molecule constituted by unmodified nucleotides, the 3' position of one of two adjacent unmodified nucleotides is usually linked to the 5' position of the other unmodified nucleotide through a phosphodiester bond. In one embodiment, the unmodified nucleotide is an unmodified deoxyribonucleotide, and the unmodified nucleic acid molecule is composed of unmodified deoxyribonucleotides.

The modified nucleotide refers to a nucleotide containing a chemical modification to the unmodified nucleotide. The modified nucleotide may be artificially synthesized or may occur naturally. The modified nucleotide includes a nucleotide modified at its nucleobase, sugar, backbone (internucleotide bond), 5' end and/or 3' end. The modified nucleotide also includes a nucleotide modified at any one of these sites as well as a nucleotide modified at two or more of the sites.

Examples of modified nucleic acid bases include, but are not limited to, xanthine, hypoxanthine, 2-aminoadenine, 6-thioguanine, 6-methyl derivatives and other alkyl derivatives of adenine and guanine, universal bases, 2-propyl derivatives and other alkyl derivatives of adenine and guanine, 5-halouracil and 5-halocytosine, 5-propynyluracil and 5-propynylcytosine, 5-bromouracil and 5-bromocytosine, 5-iodouracil and 5-iodocytosine, 2-thiouracil, 2-thiothymine, 5-methyl-2-thiocytosine and 2-thiocytosine, dihydrouracil, dihydrocytosine and dihydrothymine, 6-azouracil, 6-azocytosine and 6-azothymine, 5-uracil (pseudouracil), 4-thiouracil, 8-halo, amino, thiol, thioalkyl, hydroxyl and other 8-substituted adenine and guanine, 5-trifluoromethyl and other 5-substituted uracil and 5-substituted cytosine, 7-methylguanine, deazapurines (e.g., 7-deazaguanine, 7-deaza-8-azaguanine, 7-substituted-7-deazaguanine, 7-substituted-7-deaza-8-azaguanine and corresponding deazaadenine), heterocyclic substituted analogs of purines and pyrimidines, such as aminoethyl oxyphenoxazine, derivatives of purines and pyrimidines (e.g., 1-alkyl derivatives, 1-alkenyl derivatives, heteroaromatic derivatives and 1-alkynyl derivatives) and their tautomers, 8-oxo-N6-methyladenine, 7-diazaxanthine, 5-alkylcytosines such as 5-methylcytosine, 5-alkyluracils such as 5-ethyluracil, 3,N4-ethenocytosine, non-purine bases and non-pyrimidine bases, such as 2-aminopyridine and triazine, abasic nucleotides, deoxyabasic nucleotides, inverted abasic nucleotides, inverted deoxyabasic nucleotides, and the like.

Modifications to sugars include, but are not limited to, modifications at 2'-position such as 2'-O-alkyl modifications (e.g., 2'-O-methyl, 2'-O-ethyl, 2'-O-propyl modifications), 2'-methoxyethoxy modifications, 2'-deoxy modifications, 2'-halogen modifications (2'-fluoro, 2'-chloro, 2'-bromo modifications), 2'-O-allyl modifications, 2'-O-carbamoylethyl modifications, 2'-amino modifications, 2'-S-alkyl modifications, 2'-O-[2(methylamino)-2-oxoethyl] modifications, 2'-propargyloxy, 2'-O-(N-methylcarbamate) modifications, 2'-O-(2,4-dinitrophenyl) modifications, 2'-deoxy-2'-fluoro-β-D-arabino modifications, modifications at 4'-position such as 4'-thio modifications, 4'-C-hydroxymethyl modifications, and others like ethynyl, ethenyl, propenyl, CF, cyano, imidazole, carboxylate, thioate, C₁-C₁₀ lower alkyl, substituted lower alkyl, alkaryl or aralkyl, OCF₃, OCN, O-, S- or N-alkyl, O-, S- or N-alkenyl, SOCH₃, SO₂CH₃, ONO₂, NO₂, N₃, heterocycloalkyl, heterocycloalkaryl, aminoalkylamino, polyalkylamino or substituted silyl. Other modified sugars include, for example, locked nucleic acids (LNA), oxetane-LNA (OXE), unlocked nucleic acids (UNA), ethylene-bridged nucleic acids (ENA), altritol nucleic acids (ANA), hexitol nucleic acids (HNA), methylated LNA (cEt), amide-bridged nucleic acids (AmNA), guanidine-bridged nucleic acids (GuNA), tricyclic bridged nucleic acids (TriCyclo-DNA), spirocyclopropylene bridged nucleic acids (scpBNA), and 5'-cyclopropylene modified nucleic acids (see WO2020158910).

In the present disclosure, alkyl group includes saturated aliphatic groups, including straight-chain alkyl groups (e.g., methyl, ethyl, propyl, butyl, pentyl, hexyl, heptyl, octyl, nonyl, decyl, etc.), branched-chain alkyl groups (isopropyl, tert-butyl, isobutyl, etc.), cycloalkyl (alicyclic) groups (cyclopropyl, cyclopentyl, cyclohexyl, cycloheptyl, cyclooctyl), and alkyl substituted cycloalkyl groups. In certain embodiments, a straight chain or branched chain alkyl has 6 or fewer carbon atoms in its backbone (e.g., C₁-C₆ for straight chain, C₃-C₆ for branched chain), and more preferably 4 or fewer. Likewise, preferred cycloalkyls may have from 3-8 carbon atoms in their ring structure, and more preferably have 5 or 6 carbons in the ring structure. The term C₁-C₆ includes alkyl groups containing 1 to 6 carbon atoms. The alkyl group can be substituted alkyl group such as alkyl moieties having substituents replacing hydrogen on one or more carbons of the hydrocarbon backbone. Such substituents can include, e.g., alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

In the present disclosure, alkoxy group includes substituted and unsubstituted alkyl, alkenyl, and alkynyl groups covalently linked to an oxygen atom. Examples of alkoxy groups include methoxy, ethoxy, isopropyloxy, propoxy, butoxy, and pentoxy groups. Examples of substituted alkoxy groups include halogenated alkoxy groups. The alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Examples of halogen substituted alkoxy groups include, but are not limited to, fluoromethoxy, difluoromethoxy, trifluoromethoxy, chloromethoxy, dichloromethoxy, trichloromethoxy, etc.

In the present disclosure, halogens include fluorine, bromine, chlorine, iodine.

Modified backbones include, but are not limited to, phosphorothioate, thiophosphate, phosphoroselenoate, selenophosphate, carbophosphonate, diphosphate diester, triazole, dialkyl sulfide, boronate, piperazine, guanidine, amide, urea, morpholino phosphoramidate, morpholino phosphorodiamidate, S-methylthiourea, thioribose-D-phosphate, 2'-5' linkage (also referred to as 5'-2' or 2'5' nucleotide or 2'5' ribonucleotide), PACE, PNA, 3'-(or-5') deoxy-3'-(or-5') thio-phosphorothioate, phosphorodithioate, 3'-(or-5') deoxyphosphinate, boranophosphate, 3'-(or-5') deoxy-3'-(or 5'-) aminophosphoramidate, hydrogen phosphonate, phosphonate, boranophosphate ester, phosphoramidate, N-sulfonyl phosphoramidate, alkyl or aryl phosphonate and phosphotriester modifications, alkyl phosphotriester, phosphotriester linkage, 5'-ethoxyphosphodiester, P-alkyloxyphosphotriester, methylphosphonate, morpholino, and non-phosphorus-containing linkages such as carbonate, carbamate, silyl, sulfur, sulfonate, sulfonamide, formaldehyde acetal, thioformaldehyde acetal, oxime, methyleneimino, methylenemethylimino, methylenehydrazo, methylenedimethylhydrazo, and methyleneoxymethylimino.

Examples of 5'- and/or 3'- end modification include addition of a capping moiety to 5'- and/or 3'- end, and modification at terminal phosphate groups, such as [3'-3']-inverted deoxyribose, deoxyribonucleotide, [5'-3']-3'-deoxyribonucleotide, [5'-3']-ribonucleotide, [5'-3']-3'-O-methyl ribonucleotide, 3'-glyceryl, [3'-5']-3'-deoxyribonucleotide, [3'-3']-deoxyribonucleotide, [5'-2']-deoxyribonucleotide, and [5'-3']-dideoxyribonucleotide. Non-limiting examples of capping moiety include an abasic nucleotide, a deoxy abasic nucleotide, an inverted (deoxy) abasic nucleotide, a hydrocarbon (alkyl) moiety and derivatives thereof, a mirror nucleotide (L-DNA or L-RNA), bridged nucleic acids including LNA and ethylene bridged nucleic acids, linkage modified nucleotides (e.g. PACE) and base modified nucleotides, glyceryl, dinucleotide, acyclic nucleotide, amino, fluoro, chloro, bromo, CN, CF, methoxy, imidazole, carboxylate, thioate, C₁ to C₁₀ lower alkyl, substituted lower alkyl, alkaryl or aralkyl, OCF₃, OCN, O , S , or N-alkyl, O , S , or N-alkenyl, SOCH₃, SO₂CH₃, ONO₂, NO₂, N₃, heterocycloalkyl, heterocycloalkaryl, aminoalkylamino, polyalkylamino or substituted silyl, or polyethylene glycol. The capping moiety may serve as a non-nucleotide overhang.

The ASO of the present disclosure may have functional moieties added. Such functional moieties include, for example, cell membrane permeation-enhancing moieties, expression inhibition-enhancing moieties, and targeting moieties.

Examples of cell membrane permeation-enhancing moieties include various cell-penetrating peptides (CPP). Non-limiting examples of the CPP include, TAT, polyarginine (R5, R8, R9, etc.), Penetratin, Pep-1, proline-rich peptide (Pro), TAT-HA2, Hph-1, HP4, LAH4, LAH4-L1, Vectofusin-1, low-molecular-weight protamine (LMWP), LL-37, Pep-7, Pept1, Pept2, IVV-14, Transportant, Ig(v), pVEC, HRSV, TGN, Derived Ku-70, RW(n), RRRRRRGGRRRRG (SEQ ID NO:486), SVS-1, L-CPP, RLW, K16ApoE, Angiopep-2, ACPP, KAFAK, hCT(9-32), VP22, and the like (see, e.g., Vanova et al., Materials (Basel). 2019;12(17). pii: E2671, Silva et al., Biomolecules. 2019;9(1). pii: E22, Derakhshankhah and Jafari, Biomed Pharmacother. 2018;108:1090-1096).

Examples of expression inhibition-enhancing moieties include RNA molecules complementary to ASO (Nishina et al., Nat Commun. 2015;6:7969) or DNA molecules (Asami et al., Mol Ther. 2021;29(2):838-847), etc. These RNA or DNA molecules may be integrated with ASO to form a single strand as a whole (WO2019/022196) or may hybridize with ASO as separate molecules.

Examples of targeting moieties include various targeting molecules such as GalNAc, mannose, GLP-1, neurotensin, antibodies, cholesterol, tocopherol (e.g., α-tocopherol), and their derivatives (e.g., eGLP1). In some embodiments, the targeting molecules may target cells expressing Hic-5, such as fibroblasts of various organs like cardiac fibroblasts, pulmonary fibroblasts, hepatic stellate cells, pancreatic stellate cells, myofibroblasts, CAF, tumor cells, etc. Such targeting molecules include, for example, cyclic RGD peptides, vitamin A, cholesterol, hyaluronic acid, mannose-6-phosphate, anti-synaptophysin antibodies, etc. (Chen et al., J Pharmacol Exp Ther. 2019;370(3):695-702, Ansboro et al., Eur Cell Mater. 2012;23:310-8, etc.).

Non-limiting examples of modified nucleotides are also stated, for example, in Deleavey and Damha, Chem Biol. 2012;19(8):937-54, Freier and Altmann, Nucleic Acids Res, 1997;25(22)4429-43, Wan and Seth J Med Chem 2016; 59, 9645-67, Hall RNA 2023; 29 423-33, Clave et al., RSC Chem Biol. 2020;2(1):94-150, Ochoa and Milam, Molecules. 2020;25(20):4659, etc.

In some embodiments, the ASO of the present disclosure has modifications on the nucleotides at the 5' end and/or 3' end (preferably on their sugar moieties). In a preferred embodiment, the ASO of the present disclosure has modifications on the nucleotides at both the 5' end and 3' end (preferably on their sugar moieties). In certain embodiments, the ASO of the present disclosure has modifications on at least one, preferably 2 to 4, of the nucleotides from the 1^{st} to the 6^{th} position from the 5' end and/or at least one, preferably 2 to 4, of the nucleotides from the 1^{st} to the 6^{th} position from the 3' end. The position and number of modifications may be the same or different between the 5' end and the 3' end. In some specific embodiments, the ASO of the present disclosure has modifications on the nucleotides (preferably on their sugar moieties) at the 1^{st} to 2^{nd}, 1^{st} to 3^{rd}, 1^{st} and 4^{th}, 1^{st} and 5^{th}, 1^{st} and 3^{rd} to 4^{th}, 1^{st} to 2^{nd} and 4^{th}, 1^{st} and 4^{th} to 5^{th} 1^{st}, 3^{rd} and 5^{th}, 1^{st} and 5^{th} to 6^{th}, 1^{st} and 3^{rd} to 5^{th}, 1^{st} to 2^{nd} and 4^{th} to 5^{th}, 1^{st} to 3^{rd} and 5^{th}, 1^{st} and 6^{th} positions from the 5' end. In some specific embodiments, the ASO of the present disclosure has modifications on the nucleotides (preferably their sugar moieties) at the 1^{st} to 2^{nd}, 1^{st} to 3^{rd} 1^{st} and 3^{rd}, 1^{st} to 2^{nd} and 4^{th} positions from the 3' end. In a preferred embodiment, the sugar moiety modifications of the nucleotides at the 5' end and/or 3' end are selected from LNA, 2'-MOE, and 2'-OMe.

In a preferred specific embodiment, the ASO of the present disclosure has modifications on the nucleotides (preferably their sugar moieties) at the 1^{st} to 2^{nd} positions from the 5' end and 1^{st} to 2^{nd} positions from the 3' end, 1^{st} to 3^{rd} positions from the 5' end and 1^{st} to 3^{rd} positions from the 3' end, 1^{st} and 4^{th} positions from the 5' end and 1^{st} to 2^{nd} positions from the 3' end, 1^{st} and 3^{rd} to 4^{th} positions from the 5' end and 1^{st} to 2^{nd} positions from the 3' end, 1^{st} to 2^{nd} and 4^{th} positions from the 5' end and 1^{st} to 2^{nd} positions from the 3' end, 1^{st} to 2^{nd} and 4^{th} positions from the 5' end and 1^{st} to 3^{rd} positions from the 3' end, 1^{st} to 2^{nd} and 4^{th} positions from the 5' end and 1^{st} to 2^{nd} and 4^{th} positions from the 3' end, 1^{st} and 3^{rd} to 4^{th} positions from the 5' end and 1^{st} to 3^{rd} positions from the 3' end, 1^{st} and 4^{th} positions from the 5' end and 1^{st} to 2^{nd} and 4^{th} positions from the 3' end, 1^{st} and 3^{rd} to 4^{th} positions from the 5' end and 1^{st} to 2^{nd} and 4^{th} positions from the 3' end, 1^{st} and 4^{th} to 5^{th} positions from the 5' end and 1^{st} to 2^{nd} positions from the 3' end, 1^{st}, 3^{rd} and 5^{th} positions from the 5' end and 1^{st} to 3^{rd} positions from the 3' end, 1^{st} and 4^{th} to 5^{th} positions from the 5' end and 1^{st} to 3^{rd} positions from the 3' end, 1^{st} and 5^{th} to 6^{th} positions from the 5' end and 1^{st} to 2^{nd} positions from the 3' end, 1^{st} and 5^{th} positions from the 5' end and 1^{st} to 3^{rd} positions from the 3' end, 1^{st} and 3^{rd} to 5^{th} positions from the 5' end and 1^{st} and 3^{rd} positions from the 3' end, 1^{st} and 3^{rd} positions from the 5' end and 1^{st} to 2^{nd} and 4^{th} positions from the 3' end, 1^{st} to 2^{nd} positions from the 5' end and 1^{st} to 2^{nd} and 4^{th} positions from the 3' end, 1^{st} and 3^{rd} positions from the 5' end and 1^{st} to 3^{rd} positions from the 3' end, 1^{st} and 4^{th} positions from the 5' end and 1^{st} to 3^{rd} positions from the 3' end, 1^{st}, 3^{rd} and 5^{th} positions from the 5' end and 1^{st} to 2^{nd} positions from the 3' end, 1^{st} to 3^{rd} positions from the 5' end and 1^{st} to 2^{nd} and 4^{th} positions from the 3' end, 1^{st} and 4^{th} to 5^{th} positions from the 5' end and 1^{st} and 3^{rd} positions from the 3' end, 1^{st} and 3^{rd} to 4^{th} positions from the 5' end and 1^{st} and 3^{rd} positions from the 3' end, 1^{st} to 2^{nd} and 4^{th} positions from the 5' end and 1^{st} and 3^{rd} positions from the 3' end, 1^{st} and 3^{rd} to 5^{th} positions from the 5' end and 1^{st} to 3^{rd} positions from the 3' end, 1^{st} to 2^{nd} and 4^{th} to 5^{th} positions from the 5' end and 1^{st} to 3^{rd} positions from the 3' end, 1^{st} to 3^{rd} and 5^{th} positions from the 5' end and 1^{st} to 3^{rd} positions from the 3' end, or 1^{st} and 5^{th} positions from the 5' end and 1^{st} to 2^{nd} positions from the 3' end.

The structures of the ASO of the present disclosure containing modified nucleotides are illustrated in Table 2.

**[Table 2-1]**

| Table 2 Exemplary ASO Structures (1) | | | |
|---|---|---|---|
| ASO Number | Sequence | LNA Position | SEQ ID NO |
| M1 | TGTGTGAAGTGGTGGT | 1-3, 14-16 | 313 |
| M2 | ATGTGTGAAGTGGTGG | 1-3, 14-16 | 314 |
| M3 | CATGTGTGAAGTGGTG | 1-3, 14-16 | 315 |
| M4 | TGGCCATAGGGTGG | 1-2, 13-14 | 316 |
| M5 | GTGGCCATAGGGTG | 1-2, 13-14 | 317 |
| M6 | TGTGCTGTATAGATGA | 1-3, 14-16 | 318 |
| M7 | AACTGAGACATGATTT | 1-3, 14-16 | 319 |
| M8 | GAACTGAGACATGATT | 1-3, 14-16 | 320 |
| M9 | GGAACTGAGACATGAT | 1-3, 14-16 | 321 |
| M10 | GGGAACTGAGACATGA | 1-3, 14-16 | 322 |
| M11 | TGGGAACTGAGACATG | 1-3, 14-16 | 323 |
| M12 | ATGGGAACTGAGACAT | 1-3, 14-16 | 324 |
| M13 | GATGGGAACTGAGACA | 1-3, 14-16 | 325 |
| M14 | AGATGGGAACTGAGAC | 1-3, 14-16 | 326 |
| M15 | TAGATGGGAACTGAGA | 1-3, 14-16 | 327 |
| M16 | GGGTGAGCTCTTGTCT | 1-3, 14-16 | 328 |
| M17 | TGGGTGAGCTCTTGTC | 1-3, 14-16 | 329 |
| M18 | GTGGGTGAGCTCTTGT | 1-3, 14-16 | 330 |
| M19 | AGTGGGTGAGCTCTTG | 1-3, 14-16 | 331 |
| M20 | CAGTGGGTGAGCTCTT | 1-3, 14-16 | 332 |
| M21 | CTGAAGGCTTTGAGGG | 1-3, 14-16 | 333 |
| M22 | GCTGAAGGCTTTGAGG | 1-3, 14-16 | 334 |
| M23 | GGCTGAAGGCTTTGAG | 1-3, 14-16 | 335 |
| M24 | TGGCTGAAGGCTTTGA | 1-2, 15-16 | 336 |
| M25 | TTCCTGAGTGGCGGAG | 1-2, 15-16 | 337 |
| M26 | TCCTGAGTGGCGGA | 1-2, 13-14 | 338 |
| M27 | TTCCTGAGTGGCGG | 1-3, 12-14 | 339 |
| M28 | CAGTTCCTGAGTGGCG | 1-3, 14-16 | 340 |
| M29 | CCAGTTCCTGAGTGGC | 1-3, 14-16 | 341 |
| M30 | TCCAGTTCCTGAGTGG | 1-3, 14-16 | 342 |
| M31 | ATCCAGTTCCTGAGTG | 1-3, 14-16 | 343 |
| M32 | TATCCAGTTCCTGAGT | 1-3, 14-16 | 344 |
| M33 | TTGCCCAGCTATAGGT | 1-3, 14-16 | 345 |
| M34 | CTTGCCCAGCTATAGG | 1-3, 14-16 | 346 |
| M35 | ACTTGCCCAGCTATAG | 1-3, 14-16 | 347 |
| M36 | TTTTGTGTCGGATGGG | 1-3, 14-16 | 348 |
| M37 | ATTTTGTGTCGGATGG | 1-3, 14-16 | 349 |
| M38 | CATTTTGTGTCGGATG | 1-3, 14-16 | 350 |
| M39 | CCATTTTGTGTCGGAT | 1-3, 14-16 | 351 |
| M40 | ACCATTTTGTGTCGGA | 1-3, 14-16 | 352 |
| M41 | AGCATGGAAATGGTTT | 1-3, 14-16 | 353 |
| M42 | GAGCATGGAAATGGTT | 1-3, 14-16 | 354 |
| M43 | TGAGCATGGAAATGGT | 1-3, 14-16 | 355 |
| M44 | CTGAGCATGGAAATGG | 1-3, 14-16 | 356 |
| M45 | GCTGAGCATGGAAATG | 1-3, 14-16 | 357 |

**[Table 2-2]**

| | | | |
|---|---|---|---|
| M46 | AGCCTTTGGTGAGTGG | 1-3, 14-16 | 358 |
| M47 | GAGCCTTTGGTGAGTG | 1-3, 14-16 | 359 |
| M48 | GGAGCCTTTGGTGAGT | 1-3, 14-16 | 360 |
| M49 | AGGAGCCTTTGGTGAG | 1-3, 14-16 | 361 |
| M50 | AAGGAGCCTTTGGTGA | 1-3, 14-16 | 362 |
| M51 | TTTTGAAGGCTTAGTT | 1-3, 14-16 | 363 |
| M52 | TTTTTGAAGGCTTAGT | 1-3, 14-16 | 364 |
| M53 | GTTTTTGAAGGCTTAG | 1-3, 14-16 | 365 |
| M54 | TGTTTTTGAAGGCTTA | 1-3, 14-16 | 366 |
| M55 | GTGTTTTTGAAGGCTT | 1-3, 14-16 | 367 |
| M56 | GCGGACAGCTTGGG | 1-2, 13-14 | 368 |
| M57 | GGCGGACAGCTTGG | 1-2, 13-14 | 369 |
| M58 | TGGCGGACAGCTTG | 1-2, 13-14 | 370 |
| M59 | CTGGCGGACAGCTT | 1-2, 13-14 | 371 |
| M60 | GCTGGCGGACAGCT | 1-2, 13-14 | 372 |
| M61 | TGGCGGTGCGCTGG | 1-2, 13-14 | 373 |
| M62 | ATGGCGGTGCGCTG | 1-2, 13-14 | 374 |
| M63 | CATGGCGGTGCGCT | 1-2, 13-14 | 375 |
| M64 | CCATGGCGGTGCGC | 1-2, 13-14 | 376 |
| M65 | TCCATGGCGGTGCG | 1-2, 13-14 | 377 |
| M66 | CTCCATGGCGGTGC | 1-2, 13-14 | 378 |
| M67 | GGCCATAGGGTGGG | 1-2, 13-14 | 379 |
| M68 | CTGAGTGGCGGAGG | 1-2, 13-14 | 380 |
| H1 | AGAGGAGAATGGAGGG | 1-3, 14-16 | 381 |
| H2 | AAGAGGAGAATGGAGG | 1-3, 14-16 | 382 |
| H3 | GAAGAGGAGAATGGAG | 1-3, 14-16 | 383 |
| H4 | GTCTTTTCTTATCTTC | 1-3, 14-16 | 384 |
| H5 | GGTCTTTTCTTATCTT | 1-3, 14-16 | 385 |
| H6 | GGGTCTTTTCTTATCT | 1-3, 14-16 | 386 |
| H7 | TGGGTCTTTTCTTATC | 1-3, 14-16 | 387 |
| H8 | CTGGGTCTTTTCTTAT | 1-3, 14-16 | 388 |
| H9 | GAAGTCAGAGAGTGAG | 1-3, 14-16 | 389 |
| H10 | GGAAGTCAGAGAGTGA | 1-3, 14-16 | 390 |
| H11 | CGGAAGTCAGAGAGTG | 1-3, 14-16 | 391 |
| H12 | GCGGAAGTCAGAGAGT | 1-3, 14-16 | 392 |
| H13 | CGCGGAAGTCAGAGAG | 1-3, 14-16 | 393 |
| H14 | ACGCGGAAGTCAGAGA | 1-3, 14-16 | 394 |
| H15 | AACGCGGAAGTCAGAG | 1-3, 14-16 | 395 |
| H16 | GAACGCGGAAGTCAGA | 1-3, 14-16 | 396 |
| H17 | TGAACGCGGAAGTCAG | 1-3, 14-16 | 397 |
| H18 | TTGAACGCGGAAGTCA | 1-3, 14-16 | 398 |
| H19 | CCCCAGCATGGTGTCT | 1-3, 14-16 | 399 |
| H20 | TGCCTCCCAGGGCGGT | 1-3, 14-16 | 400 |
| H21 | ATGGGCTGGTTGCAGA | 1-3, 14-16 | 401 |
| H22 | GATGGGCTGGTTGCAG | 1-3, 14-16 | 402 |
| H23 | CGTGGAAACCCTCATC | 1-3, 14-16 | 403 |

**[Table 2-3]**

| | | | |
|---|---|---|---|
| H24 | TCGTGGAAACCCTCAT | 1-3, 14-16 | 404 |
| H25 | CTCGTGGAAACCCTCA | 1-3, 14-16 | 405 |
| H26 | GCTCGTGGAAACCCTC | 1-3, 14-16 | 406 |
| H27 | TCCTCCATGGCGGG | 1-2, 13-14 | 407 |
| H28 | GTCCTCCATGGCGG | 1-2, 13-14 | 408 |
| H29 | GGTCCTCCATGGCG | 1-2, 13-14 | 409 |
| H30 | CGCAGGGCGCTCTT | 1-2, 13-14 | 410 |
| H31 | CCGCAGGGCGCTCT | 1-2, 13-14 | 411 |
| H32 | TCCGCAGGGCGCTC | 1-2, 13-14 | 412 |
| H33 | CTCCGCAGGGCGCT | 1-2, 13-14 | 413 |
| H34 | CCCAGCATGGTGTC | 1-2, 13-14 | 414 |
| H35 | CCCCAGCATGGTGT | 1-2, 13-14 | 415 |
| H36 | GCCCCAGCATGGTG | 1-2, 13-14 | 416 |
| H37 | AGCCCCAGCATGGT | 1-2, 13-14 | 417 |
| H38 | CCCAGGGCGGTGGA | 1-2, 13-14 | 418 |
| H39 | TCCCAGGGCGGTGG | 1-2, 13-14 | 419 |
| H40 | CTCCCAGGGCGGTG | 1-2, 13-14 | 420 |
| H41 | ATGGGCTGGTTGCA | 1-2, 13-14 | 421 |
| H42 | GATGGGCTGGTTGC | 1-2, 13-14 | 422 |
| H43 | CTCGTGGAAACCCT | 1-2, 13-14 | 423 |
| H44 | GGGTGGAAGCGGCG | 1-2, 13-14 | 424 |
| H45 | CGGGTGGAAGCGGC | 1-2, 13-14 | 425 |
| H46 | CCGGGTGGAAGCGG | 1-2, 13-14 | 426 |
| H47 | TCCGGGTGGAAGCG | 1-2, 13-14 | 427 |
| H48 | GTCCGGGTGGAAGC | 1-2, 13-14 | 428 |
| H49 | GCGCAGGCAGAAGG | 1-2, 13-14 | 429 |
| H50 | GGCGCAGGCAGAAG | 1-2, 13-14 | 430 |
| H51 | GGGCGCAGGCAGAA | 1-2, 13-14 | 431 |
| H52 | CGGGCGCAGGCAGA | 1-2, 13-14 | 432 |
| H53 | GCGGGCGCAGGCAG | 1-2, 13-14 | 433 |
| H1687 | ACCGAGGCTTGCATAC | 1,4,15-16 | 434 |
| H1687_16_32_1 | ACCGAGGCTTGCATAC | 1,3-4,15-16 | 435 |
| H1687_16_32_2 | ACCGAGGCTTGCATAC | 1-2,4,15-16 | 436 |
| H1790 | GGCATTAAGTTCCTGAA | 1-2,4,15-17 | 437 |
| H1790_18 | TGGCATTAAGTTCCTGAA | 1-2,4,15,17-18 | 438 |
| H1791 | TGGCATTAAGTTCCTGA | 1,3-4,15-17 | 439 |
| H1791_18 | GTGGCATTAAGTTCCTGA | 1,4,15,17-18 | 440 |
| H1994_18 | ACATGATTTCATCTGGAG | 1,3-4,15,17-18 | 441 |
| H1995_18 | GACATGATTTCATCTGGA | 1,4-5,16-18 | 442 |
| H1996_18 | AGACATGATTTCATCTGG | 1-2,4,15,17-18 | 443 |
| H2002_18 | GAACTGAGACATGATTTC | 1,3,5,16-18 | 444 |
| H2003_18 | GGAACTGAGACATGATTT | 1,4-5,16-18 | 445 |
| H2004_18 | GGGAACTGAGACATGATT | 1,5-6,17-18 | 446 |
| H2005_18 | TGGGAACTGAGACATGAT | 1,3,5,16-18 | 447 |
| H2007_18 | GATGGGAACTGAGACATG | 1,5,16-18 | 448 |
| H2008_18 | AGATGGGAACTGAGACAT | 1-2,4,15,17-18 | 449 |

**[Table 2-4]**

| | | | |
|---|---|---|---|
| H2010_18 | CTAGATGGGAACTGAGAC | 1,3-4,15,17-18 | 450 |
| H2576_18 | AGGTTTATTGCAGGAGCC | 1,4,15,17-18 | 451 |
| H2577 | TAGGTTTATTGCAGGAGC | 1,3-5,16,18 | 452 |
| H2577_17 | AGGTTTATTGCAGGAGC | 1,3,14,16-17 | 453 |
| H2578_18 | ATAGGTTTATTGCAGGAG | 1,3-4,15,17-18 | 454 |
| H26_1 | GCTCGTGGAAACCCTC | 1-2,13,15-16 | 455 |
| H26_2 | GCTCGTGGAAACCCTC | 1,3,14-16 | 456 |
| H26_3 | GCTCGTGGAAACCCTC | 1,4,15-16 | 457 |
| H26_cho | GCTCGTGGAAACCCTC | 1-3,14-16 | 458 |
| H26_toco | GCTCGTGGAAACCCTC | 1-3,14-16 | 459 |
| H26A4_1 | CGCTCGTGGAAACCCT | 1-2,13,15-16 | 460 |
| H26A4_2 | CGCTCGTGGAAACCCT | 1-3,14-16 | 461 |
| H26A4_3 | CGCTCGTGGAAACCCT | 1,3-4,15-16 | 462 |
| H26A5_1 | CGCTCGTGGAAACCCTC | 1,3,14,16-17 | 463 |
| H26A5_2 | CGCTCGTGGAAACCCTC | 1,4,15-17 | 464 |
| H26A5_3 | CGCTCGTGGAAACCCTC | 1,3,5,16-17 | 465 |
| H3974_1 | GCGCTCAAAGTAGCACTC | 1,5-6,17-18 | 466 |
| H3974_17 | CGCTCAAAGTAGCACTC | 1-3,14,16-17 | 467 |
| H3974_2 | GCGCTCAAAGTAGCACTC | 1,4-5,16,18 | 468 |
| H3975 | GCGCTCAAAGTAGCACT | 1,3-4,15,17 | 469 |
| H3975_18 | AGCGCTCAAAGTAGCACT | 1,4-5,16-18 | 470 |
| H3976 | AGCGCTCAAAGTAGCAC | 1-2,4,15,17 | 471 |
| H3976_17 | AGCGCTCAAAGTAGCAC | 1,3-5,16-17 | 472 |
| H3977_18 | GAAGCGCTCAAAGTAGCA | 1,3-4,15,17-18 | 473 |
| H3978_17 | GAAGCGCTCAAAGTAGC | 1,3-4,15-17 | 474 |
| H3979_18 | GAGAAGCGCTCAAAGTAG | 1,3,5,16-18 | 475 |
| H3979_18_33_1 | GAGAAGCGCTCAAAGTAG | 1,3-4,15,17-18 | 476 |
| H3979_18_33_2 | GAGAAGCGCTCAAAGTAG | 1-2,4,15,17-18 | 477 |
| H3979_18_43_1 | GAGAAGCGCTCAAAGTAG | 1,3-5,16-18 | 478 |
| H3979_18_43_2 | GAGAAGCGCTCAAAGTAG | 1-2,4-5,16-18 | 479 |
| H3979_18_43_3 | GAGAAGCGCTCAAAGTAG | 1-3,5,16-18 | 480 |
| H3980_17 | GAGAAGCGCTCAAAGTA | 1,3-5,16-17 | 481 |
| H4745_18 | CAGCTGCAGGAAGTCCCG | 1,6,16-17 | 482 |
| H4800_18 | CCGAGATGTAGTTATCCA | 1,5,16-18 | 483 |
| H4801_17 | CCGAGATGTAGTTATCC | 1,3,5,16-17 | 484 |
| H4802_16 | CCGAGATGTAGTTATC | 1-3,14-16 | 485 |

In the table, H26_cho is modified at the 5' end of H26 with TEG-cholesterol, and H26_toco is modified at the 5' end of H26 with tocopherol. The nucleotides of each ASO are all deoxyribonucleotides except at the LNA positions, all inter-nucleotide linkages are phosphorothioate bonds, and all C are 5-methylcytosine.

Other structures of the ASO of the present disclosure containing modified nucleotides are illustrated in Table 3.

**[Table 3]**

| Table 3 Exemplary ASO Structures (2) | | | |
|---|---|---|---|
| ASO Number | Sequence | MOE Position | SEQ ID NO: |
| H26M1 | CGCTCGTGGAAACCCTCATC | 1-5, 16-20 | 517 |
| H26M2 | GCGCTCGTGGAAACCCTCAT | 1-5, 16-20 | 518 |
| H26M3 | CGCGCTCGTGGAAACCCTCA | 1-5, 16-20 | 519 |
| H26M4 | TCGCGCTCGTGGAAACCCTC | 1-5, 16-20 | 520 |

In the table, the nucleotides of each ASO are all deoxyribonucleotides except at the MOE positions, all inter-nucleotide linkages are phosphorothioate bonds, and all C are 5-methylcytosine.

In some embodiments, the ASO of the present disclosure is selected from the ASOs listed in Table 2. In some preferred embodiments, the ASO of the present disclosure is selected from ASO numbers H10 to H29, H31, H35, H36, H38, H39, H41, H42, and H44 to H53 (SEQ ID NOs:390 to 409, 411, 415, 416, 418, 419, 421, 422, and 424 to 433). In another preferred embodiment, the ASO of the present disclosure is selected from ASO numbers H11 to H15, H19, H25 to H31, H36, H38, and H44 to H53 (SEQ ID NOs:391 to 395, 399, 405 to 411, 416, 418, and 424 to 433). In another preferred embodiment, the ASO of the present disclosure is selected from ASO numbers H8, H13, H16 to H18, H21, and H23 to H28 (SEQ ID NOs:388, 393, 396 to 398, 401, and 403 to 408). In another preferred embodiment, the ASO of the present disclosure is selected from ASO numbers M1 to M10, M12 to M20, M22, M23, M25, M26, M29, M30, M33 to M48, M50 to M56, and M67 (SEQ ID NOs:313 to 322, 324 to 332, 334, 335, 337, 338, 341, 342, 345 to 360, 362 to 368, and 379). In another preferred embodiment, the ASO of the present disclosure is selected from ASO numbers M2 to M5, M7 to M16, M23, M28, M29, M33 to M35, M46, and M48 to M50 (SEQ ID NOs:314 to 317, 319 to 328, 335, 340, 341, 345 to 347, 358, and 360 to 362). In another preferred embodiment, the ASO of the present disclosure is selected from ASO numbers H1 to H3, H8, H10 to H18, H20 to H29, H36, H38 to H42, and H44 to H53 (SEQ ID NOs:381 to 383, 388, 390 to 398, 400 to 409, 416, 418 to 422, and 424 to 433). In another preferred embodiment, the ASO of the present disclosure is selected from ASO numbers H1687, H1790, H1790_18, H1791, H1791_18, H1994_18, H1995_18, H1996_18, H2002_18, H2003_18, H2004_18, H2005_18, H2007_18, H2008_18, H2010_18, H2576_18, H2577, H2577_17, H2578_18, H26, H26_1, H26_2, H26_3, H26A4_1, H26A4_2, H26A4_3, H26A5_1, H26A5_2, H26A5_3, H3974_1, H3974_2, H3975, H3977_18, H3978_17, H3979_18, H3980_17, H4745_18, H4801_17, and H4802_18 (SEQ ID NOs:406, 434, 437-457, 460-466, 468, 469, 473-475, 481, 482, 484, and 485). In another preferred embodiment, the ASO of the present disclosure is selected from ASO numbers H1687, H2577_17, H26, H26 cho, H26 toco, H26_1, H26_2, H26_3, H26A4_1, H26A4_2, H26A4_3, H26A5_1, H26A5_2, H26A5_3, H3979_18, H3980_17, H4745_18, H4801_17, and H4802_18 (SEQ ID NOs:406, 434, 453, 455-465, 475, 481, 482, 484, and 485). In another preferred embodiment, the ASO of the present disclosure is selected from ASO numbers H26, H3979_18_43_2, H3979_18_43_3, and H4745_18 (SEQ ID NOs:406, 479, 480, and 482). In another preferred embodiment, the ASO of the present disclosure is selected from ASO numbers H1687, H1687_16_32_1, H1687_16_32_2, H26, H26A4_3, H26A5_3_, H3979_18, H3979_18_33_1, H3979_18_33_2, H3979_18_43_1, H3979_18_43_2, H3979_18_43_3, and H4745_18 (SEQ ID NOs:406, 434-436, 462, 465, 475-480, and 482). In another preferred embodiment, the ASO of the present disclosure is selected from ASO numbers H26, H26A4_3, H26A5_3, H3979_18, H3979_18_33_1, H3979_18_43_1, H3979_18_43_2, H3979_18_43_3, and H4745_18 (SEQ ID NOs:406, 462, 465, 475, 476, 478-480, and 482). In another preferred embodiment, the ASO of the present disclosure is selected from ASO numbers M7-M9, H13, and H26 (SEQ ID NOs:319-321, 393, and 406). In another embodiment, the ASO of the present disclosure is selected from the ASOs listed in Table 3. In some preferred embodiments, the ASO of the present disclosure is selected from ASO numbers H26M2, H26M3, and H26M4.

Inhibition of Hic-5 gene expression can be evaluated, for example, by comparing the expression levels of the Hic-5 gene or protein in cells treated with the ASO of the present disclosure and untreated cells. The expression level of the Hic-5 gene can be determined by detecting the nucleic acid molecule using any known method, such as various hybridization methods, Northern blotting, Southern blotting, and various PCR methods, utilizing nucleic acids that specifically hybridize to the nucleic acid molecule encoding the gene or its unique fragment. Furthermore, the expression level of the protein can be determined by known protein detection methods, such as, but not limited to, immunoprecipitation using antibodies, EIA (enzyme immunoassay) (e.g., ELISA (enzyme-linked immunosorbent assay)), RIA (radioimmunoassay) (e.g., IRMA (immunoradiometric assay), RAST (radioallergosorbent test), RIST (radioimmunosorbent test)), Western blotting, immunohistochemistry, immunocytochemistry, flow cytometry, etc.

The ASO of the present disclosure may be delivered or administered with any known delivery carrier that assists, promotes, or facilitates delivery to the site of action, or it may be delivered or administered directly without these delivery carriers. As delivery carriers, viral vectors or non-viral vectors can be used.

Examples of the viral vector include, e.g., but are not limited to, vectors based on adenovirus, adeno-associated virus (AAV), retrovirus, vaccinia virus, poxvirus, lentivirus, and herpes virus. The viral vector may be oncolytic.

Examples of the non-viral vector include, but are not limited to, carriers in a particle form such as polymer particles, lipid particles and inorganic particles, and a bacterial vector. Nanoparticles having a nano level of size can be used as the carrier in a particle form. Examples of the polymer particles include, but are not limited to, those comprising polymers such as cationic polymers, polyamidoamine (PAMAM), chitosan, glycol chitosan, cyclodextrin, poly(lactic-co-glycolic acid) (PLGA), poly(lactic-co-caprolactonic acid) (PLCA), poly(β amino ester), atelocollagen and polyethyleneimine (PEI). The lipid particles include liposomes, non-liposomal lipid particles, and the like. The liposome is a vesicle having a lumen surrounded by a lipid bilayer, and the non-liposomal lipid particles are lipid particles having no such structure. Examples of the inorganic particles include gold nanoparticles, quantum dots, silica nanoparticles, iron oxide nanoparticles (e.g., superparamagnetic iron oxide nanoparticles (SPION)), nanotubes (e.g., carbon nanotubes (CNT)), nanodiamond, and fullerene. Examples of the bacterial vector include, but are not limited to, vectors based on Listeria bacterium, bifidus, and salmonella. The nonviral vector can be used in the delivery of not only a nucleic acid molecule but also a polypeptide such as an antibody or Cas. Various modifications can be added to the particle carrier described above. Examples thereof include stealthing with PEG, targeting with a targeting ligand, and modification with a CPP.

The ASO of the present disclosure can be administered systemically or locally to the relevant tissue ex vivo or in vivo via dermal application, transdermal application, or injection (intravenous, intradermal, subcutaneous, intramuscular, arterial, drip injection, etc.).

The ASO of the present disclosure can be delivered by a delivery system suitable for a purpose. The delivery system may include, for example, aqueous and non-aqueous gels, creams, multiple emulsions, microemulsions, liposomes, ointments, aqueous and non-aqueous solutions, lotions, aerosols, hydrocarbon bases, and powders, and may include excipients such as solubilizers, penetration enhancers (e.g., fatty acids, fatty acid esters, aliphatic alcohols, and amino acids), and hydrophilic polymers (e.g., polycarbophil and polyvinylpyrrolidone). In one embodiment, the pharmaceutically acceptable carrier is a liposome or a transdermal enhancer.

The delivery system may include patches, tablets, suppositories, pessaries, gels, and creams, and may include excipients such as solubilizers and enhancers (e.g., propylene glycol, bile salts, and amino acids), and other vehicles (e.g., polyethylene glycol, fatty acid esters and derivatives, and hydrophilic polymers such as hydroxypropyl methylcellulose and hyaluronic acid).

Methods and systems useful for the delivery of the ASO of the present disclosure are described, for example, in Huang et al., Biomater Res. 2022;26(1):49.

Some embodiments of the present disclosure relate to compositions comprising the ASO of the present disclosure (hereinafter, may be referred to as the composition of the present disclosure). The composition of the present disclosure may include, in addition to the ASO of the present disclosure, any of the carriers, diluents, delivery vehicles, delivery systems, etc. described above. The composition of the present disclosure can be used in the treatment of a disease associated with Hic-5. Therefore, the composition of the present disclosure can be a pharmaceutical composition for use in the treatment of a disease associated with Hic-5 (hereinafter, may be referred to as the pharmaceutical composition of the present disclosure). The pharmaceutical composition of the present disclosure may include one or more pharmaceutically acceptable additives (e.g., surfactants, carriers, diluents, excipients, etc.). Pharmaceutically acceptable additives are well known in the pharmaceutical field and are described, for example, in Remington's Pharmaceutical Sciences, 18th Ed., Mack Publishing Co., Easton, PA (1990), which is incorporated herein by reference in its entirety.

In some embodiments, the ASO and pharmaceutical composition of the present disclosure can be used for the treatment of a disease associated with Hic-5. The disease associated with Hic-5 include, for example, vascular diseases, fibrotic diseases, NASH, cardiac hypertrophy, osteoarthritis, tumors, etc. Vascular diseases include, for example, arteriosclerosis, arteriovenous aneurysm, thromboembolism, etc. Fibrotic diseases include, for example, liver fibrosis, cirrhosis, vocal cord scarring, vocal cord mucosal fibrosis, laryngeal fibrosis, pulmonary fibrosis, pancreatic fibrosis, myelofibrosis, myocardial infarction, post-myocardial infarction myocardial fibrosis, myocardial fibrosis, endomyocardial fibrosis, splenic fibrosis, mediastinal fibrosis, submucosal tongue fibrosis, intestinal fibrosis (e.g., associated with inflammatory bowel disease), retroperitoneal fibrosis, uterine fibrosis, scleroderma, mammary fibrosis, and various organ fibroses, scars, and keloids. Cardiac hypertrophy includes concentric hypertrophy and eccentric hypertrophy. Osteoarthritis includes primary (idiopathic) and secondary types. Secondary osteoarthritis may be caused by conditions that alter the microenvironment of cartilage, such as significant trauma, congenital joint abnormalities, metabolic disorders (e.g., hemochromatosis, Wilson's disease), infections, endocrine diseases, neurogenic disorders, and diseases that alter the normal structure and function of hyaline cartilage (e.g., rheumatoid arthritis, gout, and chondrocalcinosis).

Further examples of diseases associated with Hic-5 include sclerosing cholangitis (e.g., primary sclerosing cholangitis (PSC)). NASH (non-alcoholic steatohepatitis) has recently come to be known as MASH (metabolic dysfunction-associated steatohepatitis).

The tumor includes benign tumor and malignant tumor (cancer). The cancer includes epithelial malignant tumor and non-epithelial malignant tumor. The tumor includes solid tumor. Solid tumor includes, but not limited to, e.g., brain tumor, head and neck tumor, breast tumor, lung tumor, esophageal tumor, thyroid tumor, stomach tumor, small intestine tumor, appendix tumor, colorectal tumor, rectal tumor, liver tumor, pancreatic tumor, gallbladder tumor, bile duct tumor, anal tumor, kidney tumor, renal pelvis and ureter tumor, bladder tumor, prostate tumor, penile tumor, testicular tumor, uterine tumor, ovarian tumor, vulvar tumor, vaginal tumor, skin tumor, fibrosarcoma, fibrous histiocytoma, lipoma, liposarcoma, rhabdomyoma, rhabdomyosarcoma, leiomyoma, leiomyosarcoma, angioma, angiosarcoma, Kaposi's sarcoma, lymphangioma, lymphangiosarcoma, synovialoma, synovial sarcoma, chondroma, chondrosarcoma, osteoma, osteosarcoma, myeloma, lymphoma and GIST. In a particular embodiment, the tumor is colorectal tumor. The tumor may be present in any site, e.g., the brain, the head and the neck, the chest, extremities, the lung, the heart, thymus gland, the esophagus, the stomach, the small intestine (the duodenum, the jejunum, and the ileum), the large intestine (the colon, the cecum, the appendix, and the rectum), the liver, the pancreas, the gallbladder, the anus, the kidney, the ureter, the bladder, the prostate, the penis, the testis, the uterus, the ovary, the vulva, the vagina, the skin, striated muscle, smooth muscle, synovium, cartilage, bone, thyroid gland, adrenal gland, peritoneum, mesentery, bone marrow, the vascular system, and the lymphatic system such as lymph nodes.

The cancer includes solid cancer. Solid cancer includes, but not limited to, e.g., brain tumor, head and neck cancer, breast cancer, lung cancer, esophageal cancer, thyroid cancer, stomach cancer, small intestine cancer, appendix cancer, colorectal cancer (CRC), rectal cancer, liver cancer, pancreatic cancer, gallbladder cancer, bile duct cancer, anal cancer, kidney cancer, renal pelvis and ureter cancer, bladder cancer, prostate cancer, penile cancer, testicular cancer, uterine cancer, ovarian cancer, vulvar cancer, vaginal cancer, skin cancer, fibrosarcoma, malignant fibrous histiocytoma, liposarcoma, rhabdomyosarcoma, leiomyosarcoma, angiosarcoma, Kaposi's sarcoma, lymphangiosarcoma, synovial sarcoma, chondrosarcoma, osteosarcoma, myeloma, lymphoma and GIST. The cancer may be present in any site, e.g., the brain, the head and the neck, the chest, extremities, the lung, the heart, thymus gland, the esophagus, the stomach, the small intestine (the duodenum, the jejunum, and the ileum), the large intestine (the colon, the cecum, the appendix, and the rectum), the liver, the pancreas, the gallbladder, the anus, the kidney, the ureter, the bladder, the prostate, the penis, the testis, the uterus, the ovary, the vulva, the vagina, the skin, striated muscle, smooth muscle, synovium, cartilage, bone, thyroid gland, adrenal gland, peritoneum, mesentery, bone marrow, the vascular system, and the lymphatic system such as lymph nodes. The cancer may be accompanied by cancer-associated fibroblasts (CAFs). In some embodiments, the cancer is primary cancer. In another embodiment, the cancer is metastatic cancer. In a particular embodiment, the cancer is colorectal cancer, particularly, primary colorectal cancer. The colorectal cancer includes adenocarcinoma, squamous cell cancer, and adenosquamous cancer. Colorectal adenocarcinoma includes papillary adenocarcinoma, tubular adenocarcinoma, poorly differentiated adenocarcinoma, mucinous cancer, signet ring cell adenocarcinoma, and medullary cancer.

In the present disclosure, the "treatment" includes every type of medically acceptable prophylactic and/or therapeutic intervention aimed at cure, transient remission or prevention, etc., of a disease. The "treatment" includes medically acceptable intervention for various purposes including, e.g., the delay or arrest of progression of a disease, the regression or disappearance of a lesion, the prevention of onset of the disease or the prevention of recurrence of the disease. Therefore, the ASO and pharmaceutical compositions can be used for the treatment and/or prevention of diseases.

The ASO or pharmaceutical compositions of the present disclosure may be administered via various routes, including both oral and parenteral, such as, without limitation, oral, buccal, intrabuccal, intravenous, intramuscular, subcutaneous, intradermal, topical, rectal, intra-arterial, portal, intraventricular, transmucosal, transdermal, intranasal, intraperitoneal, intratracheal, intrapulmonary, and intrauterine routes, and may be formulated into dosage forms suitable for each route of administration. Such dosage forms and formulation methods can be appropriately adopted from any known methods (see, for example, Remington's Pharmaceutical Sciences, 18th Ed., Mack Publishing Co., Easton, PA (1990)).

Examples of the dosage form suitable for oral administration include, but are not limited to, powders, granules, tablets, capsules, solutions, suspensions, emulsions, gels, and syrups. Examples of the dosage form suitable for parenteral administration include injections such as solution-type injections, suspension-type injections, emulsion-type injection, and injections to be prepared at the time of use. The preparation for parenteral administration may be in the form of an aqueous or non-aqueous isotonic sterile solution or suspension. Delivery to lung tissues can be performed using, e.g., the form of an aerosolized agent or a spray-dried formulation suitable for administration with an inhalation apparatus or a nebulizer.

A further aspect of the present disclosure relates to kits or packs for preparing the ASO or composition, or for use in treating diseases associated with Hic-5, which comprise the ASO or composition of the present disclosure, or components thereof, as well as the ASO or composition provided in the form of such kits or packs, or their necessary components. The components of the ASO or composition included in such kits or packs are as described above for the ASO or composition. In addition to the above, the kit may further include instructions on the preparation or use of the ASO or composition (e.g., administration methods), such as user manuals, information on websites containing usage information (e.g., URLs, two-dimensional codes), and media recording usage information, such as flexible disks, CDs, DVDs, Blu-ray discs, memory cards, USB drives, etc. Such kits or packs may include all components necessary to complete the ASO or composition, but they do not necessarily have to include all components. Therefore, the kit or pack may not include reagents or solvents that are commonly available in medical settings or laboratories, such as sterile water, saline, glucose solutions, etc.

Another aspect of the present disclosure is a method for treating diseases associated with Hic-5, which includes administering an effective amount of the ASO or pharmaceutical composition of the present disclosure to a subject in need thereof (hereinafter, may be referred to as "the treatment method of the present disclosure"). Here, an effective amount refers to an amount that, for example, prevents the onset and recurrence of the disease or cures the disease. The terms "diseases associated with Hic-5" and "treatment" in the treatment method of the present disclosure are as described above for the ASO of the present disclosure.

The specific dosage of the ASO or pharmaceutical composition administered to the subject in the treatment method can be determined by considering various conditions related to the subject requiring administration, such as the type of target, purpose of the method, treatment content, type of disease, severity of symptoms, general health condition, age, weight of the subject, gender of the subject, diet, timing and frequency of administration, concomitant medications, responsiveness to treatment, and compliance with treatment. The total daily dose of the ASO or pharmaceutical composition is not limited and may be, for example, about 1 µg to about 1000 mg/kg body weight, about 10 µg to about 100 mg/kg body weight, or about 100 µg to about 10 mg/kg body weight. Alternatively, the dosage may be calculated based on the patient's body surface area.

Routes of administration include various routes encompassing both oral and parenteral, such as oral, buccal, intrabuccal, intravenous, intramuscular, subcutaneous, intradermal, topical, rectal, intra-arterial, portal, intraventricular, transmucosal, transdermal, intranasal, intraperitoneal, intratracheal, intrapulmonary, intrathecal, and intrauterine routes.

The frequency of administration differs depending on the properties of the preparation or the composition used or the conditions of the subject as described above and may be, e.g., plural times per day (i.e., 2, 3, 4 or 5 or more times per day), once a day, every few days (i.e., every 2, 3, 4, 5, 6, or 7 days), several times a week (e.g., 2, 3, or 4 times a week), every week, or every few weeks (i.e., every 2, 3, or 4 weeks).

In the present disclosure, the term "subject" refers to any biological individual, preferably an animal, more preferably a mammal, and even more preferably a human individual. The subject may be healthy (e.g., not having a specific or any disease) or may have some disease, but when treatment of diseases related to the target nucleic acid molecule is intended, it typically refers to a subject who has or is at risk of having the disease.

Another aspect of the present disclosure relates to the use of the ASO of the present disclosure in the manufacture of a medicament for the treatment of diseases associated with Hic-5 (hereinafter, may be referred to as "the use of the present disclosure").

The terms "diseases associated with Hic-5" and "treatment" in the use of the present disclosure are as described above for the ASO of the present disclosure.

Another aspect of the present disclosure relates to a composition for use in treating sclerosing cholangitis, comprising a Hic-5 inhibitor (hereinafter, may be referred to as composition A of the present disclosure).

In the present disclosure, the Hic-5 inhibitor means a substance that inhibits the expression and/or activity of Hic-5. Examples of the substance that inhibits the expression of Hic-5 include, but are not limited to, substances that modify Hic-5 gene, substances that inhibit the transcription of Hic-5 gene, substances that degrade transcripts of Hic-5 gene, substances that inhibit the translation of Hic-5 gene, and substances that inhibit normal folding of Hic-5 gene. Preferred examples of the substance that inhibits the expression of Hic-5 include inhibitory nucleic acids and genome editing molecules, particularly, inhibitory nucleic acids and genome editing molecules targeting Hic-5. Examples of the substance that inhibits the activity of Hic-5 include, but are not limited to, substances that inhibit the interaction between Hic-5 and another molecule, e.g., substances that bind to Hic-5, particularly, antibodies against the antigen Hic-5 and their antigen binding derivatives, and Hic-5 binding antibody mimetics. Other examples of the substance that inhibits the activity of Hic-5 include dominant negative mutants of Hic-5 (Shibanuma et al., Mol Biol Cell. 2003;14(3):1158-71). The Hic-5 inhibitor according to the present disclosure also includes a nucleic acid molecule encoding the aforementioned substance that inhibits the expression and/or activity of Hic-5. The Hic-5 inhibitor in the present disclosure may be a nucleic acid molecule or a polypeptide.

The inhibitory nucleic acid molecule means a nucleic acid molecule that inhibits the expression of a target molecule and includes RNAi molecule, microRNA, microRNA mimic, piRNA (Piwi-interacting RNA), ribozyme (see, e.g., Jimenez et al., Trends Biochem Sci. 2015;40(11):648-661), antisense nucleic acids, and Bonac nucleic acids, for example. The RNAi (RNA interference) molecule means any molecule having RNAi activity and encompasses, e.g., but is not limited to, siRNA (small interfering RNA), shRNA (short hairpin RNA), and the like. The RNA interference typically refers to a phenomenon, induced by a double-stranded nucleic acid molecule, in which target RNA is degraded in a sequence-specific manner. siRNA typically refers to a small nucleic acid with an antisense strand having complementarity to a target sequence, and a sense strand having complementarity to the antisense strand, wherein both the strands at least partially form a duplex. The microRNA mimic is a nucleic acid molecule that mimics the function of endogenous microRNA, and is well known in the art (e.g., van Rooij and Kauppinen, EMBO Mol Med. 2014; 6 (7): 851-64; and Chorn et al., RNA. 2012; 18 (10): 1796-804). The microRNA mimic may contain a chemical modification, and its nucleotide sequence may be changed with respect to the endogenous microRNA. shRNA is a single-stranded RNA molecule having an antisense region and a sense region having complementarity to each other, and a loop region interposed therebetween, and assumes a hairpin-shaped three-dimensional structure formed from a duplex region by the pairing of the antisense region and the sense region.

The inhibitory nucleic acid can be prepared on the basis of information on the gene sequence, etc. of Hic-5 (also called transforming growth factor beta-1-induced transcript 1 protein (TGFB1I1), androgen receptor coactivator 55 kDa protein, androgen receptor-associated protein of 55 kDa, or ARA55). For example, the gene sequence of human Hic-5 is registered under accession number NM_001042454 (transcript variant 1), NM_015927 (transcript variant 2), NM_001164719 (transcript variant 3), and the sequences are as shown in SEQ ID NOs: 1, 3, and 5, respectively (corresponding amino acid sequences are shown in SEQ ID NOs: 2, 4, and 6). For example, the following is known as the gene sequence of nonhuman animal Hic-5. Dog: accession number XM_022419832, XM_005621231, XM_014114355 and XM_005621229. Cat: accession number XM_019820649, XM_019820647, XM_003998629, XM_006942108 and XM_019820648. Horse: accession number XM_023655571 and XM_023655570. Cow: accession number BC104510. Mouse: accession number NM_001289550. Examples of the base sequences of human Hic-5 mRNA and unspliced transcripts are shown in SEQ ID NOs:503 and 504, and examples of the base sequences of mouse Hic-5 mRNA and unspliced transcripts are shown in SEQ ID NOs:505 and 506, respectively. siRNA design methods are described, e.g., in Naito and Ui-Tei, Front Genet. 2012;3:102, and miRNA design methods are described, e.g., in Mickiewicz et al., Acta Biochim Pol. 2016;63(1):71-77.

The genome editing molecule is a molecule or a molecule set capable of modifying a genome sequence in a site-specific manner. Examples thereof include, but are not limited to, molecules based on CRISPR/Cas system, TALEN, ZFN, or meganuclease (MN). The genome editing molecule based on CRISPR/Cas system functions as a set of guide RNA and Cas (CRISPR-associated protein). Examples of Cas contained in CRISPR/Cas system include, but are not limited to, Cas9, Cas12a(Cpf1), Cas12b, Cas13, xCas9, VQR, VRER, spCas9-NG, spCas9-HF1, Cas nickase (e.g., Cas9 nickase), eSpCas9, evoCas9, HypaCas9, CjCas9, Split-Cas (e.g., Split-Cas9), dCas-BE (e.g., dCas9-BE), and the like (Broeders et al., iScience. 2019;23(1):100789). CRISPR/Cas13 can edit RNA and as such, can be used in gene silencing similar to that of RNAi.

In the present disclosure, the antibody includes a whole antibody and an antigen binding fragment thereof. The class of antibody is not particularly limited and includes IgA, IgD, IgE, IgG and IgM, including IgA1, IgA2, IgG1, IgG2a, IgG2b, IgG3 and IgG4 as subclasses. The whole antibody also includes IgNAR and heavy chain antibodies. The antigen binding fragment includes Fab, Fab', F(ab')₂, Fd, Fcab, vNAR and VHH (nanobody). The antibody against the antigen Hic-5 (anti-Hic-5 antibody) is commercially available or can be obtained by a well-known approach, e.g., by immunizing animals, e.g., mice, rats, rabbits, goats, or sheep, with Hic-5 or its immunogenic fragment, and isolating serum, or by isolating anti-Hic-5 antibody-producing cells from such immunized animals, and culturing the cells, if necessary, after immortalization. The antibody may be polyclonal or monoclonal and may be a chimeric antibody, a humanized antibody or a human antibody. Hic-5 serving as an antigen is preferably human Hic-5. Hic-5 of another animal species may be used as the antigen as long as the resulting antibody is capable of recognizing human Hic-5. In another embodiment, the antibody may be an equine antibody that recognizes equine Hic-5, a canine antibody that recognizes canine Hic-5, a feline antibody that recognizes feline Hic-5, or a bovine antibody that recognizes bovine Hic-5.

The antigen binding derivative of the antibody includes an antibody derivative having antigen binding moieties (e.g., VH region, VL region, and Fv region) of the antibody combined with other moieties (e.g., CH1 region, CH2 region, CH3 region, CL region, and Fc region) of the antibody, e.g., scFv, minibody, scFv-Fc, scFv₂ (diabody), scFv₃ (triabody), scFv₄ (tetrabody), Fv-clasp (Arimori et al., Structure. 2017;25(10):1611-1622), BIf (bispecific scFv immunofusion, Kuo et al., Protein Eng Des Sel. 2012;25(10):561-9), and a bispecific antibody. Examples of the Hic-5 binding antibody mimetic include monobody (adnectin), affibody, Affimer, affitin, Anticalin, atrimer, finomer, armadillo repeat protein, Kunitz domain, nottin, avimer, DARPin, alphabody, O body, and repebody (Simeon and Chen, Protein Cell. 2018;9(1):3-14, Yu et al., Annu Rev Anal Chem (Palo Alto Calif). 2017;10(1):293-320, Wuo and Arora, Curr Opin Chem Biol. 2018 Jun;44:16-22).

When the Hic-5 inhibitor is a polypeptide (polypeptide-type Hic-5 inhibitor, e.g., an antibody, an antigen binding derivative of the antibody, an antibody mimetic, or a dominant negative mutant), the composition A of the present disclosure may comprise a nucleic acid molecule encoding the polypeptide-type Hic-5 inhibitor instead of this inhibitor. Such a nucleic acid molecule is also included in the Hic-5 inhibitor of the present disclosure. The nucleic acid molecule encoding the polypeptide-type Hic-5 inhibitor is expressed in cells so that the Hic-5 inhibitor acts in the cells and can thereby produce the desired effect. Use of the nucleic acid molecule encoding the polypeptide-type Hic-5 inhibitor is particularly useful, e.g., when the transfer of the nucleic acid molecule into cells is easier than that of the polypeptide.

Sclerosing cholangitis is a disease characterized by sclerotic changes in the bile duct, leading to bile duct stenosis and cholestasis. Sclerosing cholangitis includes primary sclerosing cholangitis (PSC), IgG4-related sclerosing cholangitis (IgG4-SC), and secondary sclerosing cholangitis. Secondary sclerosing cholangitis can occur as a consequence of diseases such as cholangitis, bile duct stones, bile duct cancer, and ischemia.

Since Hic-5 is typically present within cells, it is preferable for the Hic-5 inhibitor in composition A disclosed herein to be delivered intracellularly. For intracellular delivery of the Hic-5 inhibitor, methods such as electroporation, calcium phosphate method, dextran method, microinjection, iTOP (D'Astolfo et al., Cell. 2015;161(3):674-690), cell-penetrating peptides (CPP), viral vectors, or non-viral vectors can be used (Bruce and McNaughton, Cell Chem Biol. 2017;24(8):924-934, Lee et al., J Control Release. 2019;313:80-95, etc.). For viral and non-viral vectors, those exemplified for the ASO disclosed herein can be used. The Hic-5 inhibitor can be delivered using delivery systems exemplified for the ASO disclosed herein.

The "treatment" in composition A disclosed herein is as described above for the ASO disclosed herein.

Another aspect of the disclosure is a method for treating sclerosing cholangitis, comprising administering a therapeutically effective amount of a Hic-5 inhibitor or a composition comprising it to a subject in need thereof (hereinafter, may be referred to as "treatment method A of the present disclosure"). Here, an effective amount refers to an amount that prevents the onset and recurrence of sclerosing cholangitis or cures the disease. The subjects in this method include, but are not limited to, those diagnosed with sclerosing cholangitis and those at risk of developing sclerosing cholangitis. The types of diseases, Hic-5 inhibitors, and treatments in treatment method A of the disclosure are as described above for composition A disclosed herein.

Another aspect of the disclosure relates to a Hic-5 inhibitor for use in treating sclerosing cholangitis. A further aspect of the disclosure relates to the use of a Hic-5 inhibitor in the treatment of sclerosing cholangitis. Yet another aspect of the disclosure relates to the use of Hic-5 inhibitors in the manufacture of pharmaceuticals for treating sclerosing cholangitis. The types of sclerosing cholangitis, Hic-5 inhibitors, and treatments in these aspects are as described above for composition A disclosed herein.

The specific dosage of composition A disclosed herein, the Hic-5 inhibitor, or a composition containing the Hic-5 inhibitor administered to a subject can be determined by considering various conditions related to the subject requiring administration, such as the purpose of the method, treatment content, type of disease, severity of symptoms, general health condition, age, weight of the subject, gender of the subject, diet, timing and frequency of administration, concomitant medications, responsiveness to treatment, and compliance with treatment. The total daily dosage of composition A disclosed herein, the Hic-5 inhibitor, or a composition containing the Hic-5 inhibitor is not limited and may be, for example, about 1 µg to about 1000 mg/kg body weight, about 10 µg to about 100 mg/kg body weight, or about 100 µg to about 10 mg/kg body weight. Alternatively, the dosage may be calculated based on the patient's surface area.

The administration routes for composition A disclosed herein, the Hic-5 inhibitor, or a composition containing the Hic-5 inhibitor include various routes encompassing both oral and non-oral routes, such as oral, buccal, intraoral, intravenous, intramuscular, subcutaneous, intradermal, topical, rectal, intra-arterial, portal vein, intraventricular, intramyocardial, intra-articular, transmucosal, transdermal, intranasal, intraperitoneal, airway, intratracheal, intrabronchial, intra-alveolar, intrapulmonary, intrahepatic, and intrauterine routes.

The frequency of administration varies depending on the properties of the formulation or composition used and the conditions of the subject as described above, but may be, for example, multiple times a day (i.e., 2, 3, 4, or 5 or more times a day), once a day, every few days (i.e., every 2, 3, 4, 5, 6, or 7 days, etc.), several times a week (e.g., 2, 3, or 4 times a week, etc.), weekly, or every few weeks (i.e., every 2, 3, or 4 weeks, etc.).

### Examples

The invention will be described in detail below with reference to examples, but the content of the invention is not limited thereto. For conciseness, the catalog numbers and manufacturers of reagents, etc., are listed only at their first occurrence.

### [Example 1] Evaluation of On-Target Activity (1)

ASOs with the structure shown in Table 2 were designed. Synthesis was outsourced to Gene Design Inc. The on-target activity of the ASOs was evaluated under the following various conditions.

### <Condition 1>

KMST-6 cells (RIKEN BRC, RCB1955) are seeded at a density of 1.0×10⁴ cells/well in a 24-well plate and incubated for 24 hours, followed by transfection with ASO (200 nM) or siRNA (20 nM) using a transfection reagent (Lipofectamine^{™} RNAiMAX Transfection Reagent, Thermo Fisher Scientific). After 24 hours of incubation, proteins are extracted with a cell lysis solution containing 2% SDS, and the expression level of Hic-5 is quantified by Western blotting using an anti-mouse Hic-5 antibody (BD Transduction Laboratories^{™}, Cat#: 611164). The results are displayed as the ratio of Hic-5 expression to GAPDH expression, with the control (transfection reagent only) set to 1 (n=1).

### <Condition 2>

Same as Condition 1, except the ASO concentration is 100 nM.

### <Condition 3>

Same as Condition 1, except the ASO concentration is 20 nM.

### <Condition 4>

VSMC cells (isolated and cultured from mouse aorta according to the method described in Kim-Kaneyama et al., J Mol Cell Cardiol. 2011;50(1):77-86) are seeded at a density of 0.7×10⁴ cells/well in a 24-well plate and incubated for 24 hours, followed by transfection with ASO (100 nM) or siRNA (20 nM) using a transfection reagent (Lipofectamine^{™} RNAiMAX Transfection Reagent). After 24 hours of incubation, RNA is extracted using the SuperPrep^{®} II Cell Lysis & RT Kit for qPCR2 (Toyobo), cDNA is synthesized, and the expression level of Hic-5 is quantified by RT-PCR using KOD SYBR^{®} qPCR Mix (Toyobo) (StepOnePlus^{™}, Thermo Fisher Scientific). The results are displayed as the ratio (mean±SEM) with the control (transfection reagent only) set to 1.

### <Condition 5>

Same as Condition 4, except human HSCs (ScienCell Research Laboratories, Cat#: 5300) are seeded at a density of 1.0×10⁴ cells/well in a poly-L-lysine-coated 24-well plate.

### <Condition 6>

Same as Condition 4, except human HSCs are seeded at a density of 2.0×10⁴ cells/well.

### <Condition 7>

Same as Condition 4, except VSMC cells are seeded at a density of 1.5×10⁴ cells/well.

### <Condition 8>

Same as Condition 4, except human HSCs are seeded at a density of 0.3×10⁴ cells/well, incubated for 24 hours, transfected with ASO (5 µM) without using a transfection reagent (siRNA (20 nM) is transfected using Lipofectamine^{™} RNAiMAX Transfection Reagent), incubated for 72 hours, and results are displayed as the ratio (mean±SD) with untreated set to 1.

### <Condition 9>

Same as Condition 8, except the siRNA concentration is 5 µM, and results are displayed as the ratio (mean±SEM) with untreated set to 1.

The following siRNA was used under conditions using human-derived cells (KMST-6, human HSCs).
Sense strand: 5'- GGACCAGUCUGAAGAUAAG -3' (SEQ ID NO:487)
Antisense strand: 5'- CUUAUCUUCAGACUGGUCC -3' (SEQ ID NO:488)

The following siRNA was used under conditions using mouse-derived cells.
Sense strand: 5'- GGACCAGUCUGAAGAUAAG -3' (SEQ ID NO:489)
Antisense strand: 5'- CUUAUCUUCAGACUGGUCC -3' (SEQ ID NO:490)

Primers used for RT-PCR are as follows.

**[Table 4]**

| Table 4 RT-PCR Primers | | | |
|---|---|---|---|
| Gene | | Sequence | Species |
| *GAPDH* | S | 5'- GCACCGTCAAGGCTGAGAAC -3' (SEQ ID NO:491) | Human |
| | AS | 5'- TGGTGAAGACGCCAGTGGA -3' (SEQ ID NO:492) | |
| *Hic-5* | S | 5'- AGTGCTACTTTGAGCGCTTCTC -3' (SEQ ID NO:493) | Human |
| | AS | 5'- GCCGAAGAGCTTCAGGAAGCAAGG -3' (SEQ ID NO:494) | |
| *GAPDH* | S | 5'- AGGTCGGTGTGAACGGATTTG -3' (SEQ ID NO:495) | Mouse |
| | AS | 5'- TGTAGACCATGTAGTTGAGGTCA -3' (SEQ ID NO:496) | |
| *Hic-5* | S | 5'- ATGTCACGGTTAGGGGCTC -3' (SEQ ID NO:497) | Mouse |
| | AS | 5'- GGCTTGCATACTGTGCTGTATAG -3' (SEQ ID NO:498) | |
| *GAPDH* | S | 5'- GGTGAAGGTCGGAGTCAACGGA -3' (SEQ ID NO:499) | rat |
| | AS | 5'- GAGGGATCTCGCTCCTGGAAGA -3' (SEQ ID NO:500) | |
| *Hic-5* | S | 5'- CCAGGACAGACCAACAAGGG -3' (SEQ ID NO:501) | rat |
| | AS | 5'- AAAAGGGAGCCCCATCCTTC -3' (SEQ ID NO:502) | |

The results are shown below.

**[Table 5]**

| Table 5 Expression Inhibition Rate (1) | | | | | |
|---|---|---|---|---|---|
| ASO | Condition 4 (n=3) | Condition 5 (n=3) | ASO | Condition 4 (n=3) | Condition 5 (n=3) |
| M1 | 0.121±0.057 | 0.083±0.033 | M36 | 0.281±0.099 | 0.295±0.051 |
| M2 | 0.323±0.035 | 0.169±0.027 | M37 | 0.231±0.074 | 0.237±0.043 |
| M3 | 0.366±0.029 | 0.168±0.013 | M38 | 0.148±0.074 | 0.687±0.200 |
| M4 | 0.317±0.115 | 0.103±0.037 | M39 | 0.254±0.027 | 0.782±0.189 |
| M5 | 0.263±0.082 | 0.137±0.030 | M40 | 0.467±0.097 | 0.787±0.141 |
| M6 | 0.049±0.019 | 0.895±0.101 | M41 | 0.153±0.033 | 0.764±0.143 |
| M7 | 0.150±0.049 | 0.052±0.022 | M42 | 0.242±0.044 | 1.073±0.155 |
| M8 | 0.171±0.058 | 0.049±0.012 | M43 | 0.141±0.035 | 0.533±0.150 |
| M9 | 0.166±0.063 | 0.049±0.012 | M44 | 0.263±0.108 | 0.447±0.060 |
| M10 | 0.526±0.019 | 0.035±0.014 | M45 | 0.262±0.121 | 0.348±0.029 |
| M11 | 0.677±0.032 | 0.076±0.014 | M46 | 0.159±0.057 | 0.040±0.014 |
| M12 | 0.162±0.038 | 0.049±0.018 | M47 | 0.335±0.173 | 0.235±0.076 |
| M13 | 0.309±0.102 | 0.033±0.005 | M48 | 0.489±0.357 | 0.067±0.030 |
| M14 | 0.183±0.049 | 0.034±0.006 | M49 | 0.598±0.422 | 0.036±0.019 |
| M15 | 0.291±0.066 | 0.042±0.010 | M50 | 0.229±0.090 | 0.101±0.010 |
| M16 | 0.495±0.291 | 0.139±0.026 | M51 | 0.196±0.097 | 0.698±0.113 |
| M17 | 0.336±0.123 | 0.293±0.025 | M52 | 0.156±0.068 | 0.655±0.140 |
| M18 | 0.263±0.090 | 0.358±0.036 | M53 | 0.088±0.033 | 0.542±0.170 |
| M19 | 0.204±0.081 | 0.407±0.040 | M54 | 0.115±0.043 | 0.459±0.148 |
| M20 | 0.336±0.170 | 0.573±0.074 | M55 | 0.159±0.090 | 0.603±0.172 |
| M21 | 0.702±0.318 | 0.529±0.031 | M56 | 0.413±0.033 | 0.414±0.149 |
| M22 | 0.417±0.182 | 0.259±0.031 | M57 | 0.633±0.064 | 0.230±0.096 |
| M23 | 0.493±0.213 | 0.057±0.014 | M58 | 0.893±0.165 | 0.695±0.261 |
| M24 | 0.905±0.199 | 0.221±0.018 | M59 | 1.061±0.134 | 0.617±0.243 |
| M25 | 0.257±0.042 | 0.789±0.207 | M60 | 0.788±0.047 | 0.597±0.245 |
| M26 | 0.312±0.058 | 1.059±0.217 | M61 | 0.940±0.086 | 0.292±0.144 |
| M27 | 0.667±0.307 | 0.889±0.210 | M62 | 1.189±0.057 | 0.546±0.035 |
| M28 | 0.746±0.219 | 0.043±0.012 | M63 | 0.733±0.094 | 0.531±0.005 |
| M29 | 0.255±0.054 | 0.190±0.039 | M64 | 0.689±0.033 | 0.292±0.069 |
| M30 | 0.304±0.064 | 0.794±0.079 | M65 | 0.706±0.045 | 0.343±0.090 |
| M31 | 0.676±0.362 | 0.582±0.042 | M66 | 0.608±0.118 | 0.430±0.142 |
| M32 | 0.686±0.052 | 1.041±0.220 | M67 | 0.386±0.073 | 0.571±0.067 |
| M33 | 0.430±0.135 | 0.123±0.025 | M68 | 1.199±0.201 | 0.294±0.038 |
| M34 | 0.278±0.147 | 0.143±0.047 | siRNA | 0.556±0.242 | 0.194±0.042 |
| M35 | 0.314±0.082 | 0.153±0.027 | | | |

**[Table 6]**

| Table 6 Expression Inhibition Rate (2) | | | | | | | | | |
|---|---|---|---|---|---|---|---|---|---|
| ASO | Conditi on 1 | Conditi on 2 | Conditi on 3 | Condition 5 (n=3) | ASO | Conditi on 1 | Conditi on 2 | Conditi on 3 | Condition 5 (n=3) |
| H1 | 0.317 | 0.235 | 0.415 | 0.030±0.0 06 | H28 | 0.010 | 0.000 | 0.286 | 0.067±0.0 16 |
| H2 | 0.450 | 0.757 | - | 0.039±0.0 09 | H29 | 0.048 | 0.000 | - | 0.047±0.0 12 |
| H3 | 0.780 | 0.190 | 0.542 | 0.036±0.0 06 | H30 | 0.609 | - | - | 0.255±0.0 84 |
| H4 | 4.540 | - | - | 0.466±0.0 32 | H31 | 0.215 | 0.072 | - | 0.164±0.0 54 |
| H5 | 2.263 | - | - | 0.329±0.0 36 | H32 | 0.558 | - | - | 0.197±0.0 51 |
| H6 | 1.012 | 0.894 | - | 0.491±0.0 48 | H33 | 0.262 | - | - | 0.221±0.0 49 |
| H7 | 0.417 | 0.551 | - | 0.326±0.0 31 | H34 | 0.259 | - | - | 0.345±0.2 36 |
| H8 | 0.307 | 0.295 | 0.289 | 0.055±0.0 08 | H35 | 0.149 | 0.270 | - | 0.249±0.0 47 |
| H9 | 0.258 | 0.300 | 0.561 | 0.148±0.0 12 | H36 | 0.143 | 0.041 | - | 0.073±0.0 21 |
| H10 | 0.078 | 0.166 | 1.047 | 0.044±0.0 04 | H37 | 0.358 | - | - | 0.213±0.0 50 |
| H11 | 0.000 | 0.058 | 0.399 | 0.033±0.0 04 | H38 | 0.069 | 0.061 | - | 0.050±0.0 13 |
| H12 | 0.000 | 0.039 | 0.528 | 0.049±0.0 09 | H39 | 0.003 | 0.125 | - | 0.032±0.0 09 |
| H13 | 0.104 | 0.029 | 0.191 | 0.066±0.0 11 | H40 | 0.593 | - | - | 0.036±0.0 15 |
| H14 | 0.155 | 0.000 | - | 0.043±0.0 04 | H41 | 0.090 | 1.008 | - | 0.038±0.0 08 |
| H15 | 0.016 | 0.102 | 0.317 | 0.054±0.0 01 | H42 | 0.048 | 0.506 | - | 0.058±0.0 19 |
| H16 | 0.000 | 0.119 | 0.072 | 0.027±0.0 04 | H43 | 0.322 | - | - | 0.297±0.1 36 |
| H17 | 0.018 | 0.281 | 0.000 | 0.040±0.0 07 | H44 | 0.033 | - | - | 0.106±0.0 70 |
| H18 | 0.103 | 0.175 | 0.000 | 0.033±0.0 05 | H45 | 0.034 | 0.000 | - | 0.011±0.0 03 |
| H19 | 0.028 | 0.077 | 0.533 | 0.150±0.0 15 | H46 | 0.043 | 0.000 | - | 0.020±0.0 03 |
| H20 | 0.000 | 0.176 | 0.377 | 0.028±0.0 02 | H47 | 0.025 | 0.000 | - | 0.022±0.0 09 |
| H21 | 0.016 | 0.203 | 0.219 | 0.030±0.0 01 | H48 | 0.011 | 0.000 | - | 0.029±0.0 08 |
| H22 | 0.000 | 0.247 | 0.392 | 0.016±0.0 08 | H49 | 0.006 | 0.088 | - | 0.012±0.0 03 |
| H23 | 0.000 | 0.120 | 0.136 | 0.021±0.0 05 | H50 | 0.063 | 0.000 | - | 0.013±0.0 04 |
| H24 | 0.079 | 0.122 | 0.246 | 0.066±0.0 18 | H51 | 0.068 | 0.000 | - | 0.013±0.0 04 |
| H25 | 0.038 | 0.041 | 0.017 | 0.015±0.0 01 | H52 | 0.005 | 0.000 | 0.525 | 0.022±0.0 09 |
| H26 | 0.018 | 0.000 | 0.054 | 0.007±0.0 02 | H53 | 0.006 | 0.000 | 0.891 | 0.013±0.0 05 |
| H27 | 0.015 | 0.000 | 0.087 | 0.142±0.0 33 | siRN A | 0.218 | 0.118 | - | 0.146±0.0 11 |

**[Table 7]**

| Table 7 Expression Inhibition Rate (3) | |
|---|---|
| ASO | Condition 4 (n=8) |
| H11 | 0.921±0.190 |
| H13 | 0.373±0.072 |
| H26 | 0.255±0.042 |
| siRNA | 0.191±0.061 |

**[Table 8]**

| Table 8 Expression Inhibition Rate (4) | | | | | |
|---|---|---|---|---|---|
| ASO | Condition 6 (n=3) | Condition 8 (n=2) | ASO | Condition 6 (n=3) | Condition 8 (n=2) |
| H1687 | 0.489±0.090 | 0.433±0.157 | H3975 18 | 0.615±0.074 | - |
| H1790 | 0.350±0.049 | 0.949±0.043 | H3976 17 | 0.759±0.056 | - |
| H1791 | 0.399±0.026 | 0.903±0.094 | H3977 18 | 0.442±0.087 | 0.905±0.077 |
| H2577 | 0.344±0.038 | 0.804±0.212 | H3978 17 | 0.400±0.064 | 0.838±0.089 |
| H3974_1 | 0.325±0.039 | 0.811±0.179 | H3979_18 | 0.341±0.107 | 0.441±0.128 |
| H3974_2 | 0.487±0.056 | 0.858±0.063 | H3980_17 | 0.337±0.086 | 0.439±0.038 |
| H3975 | 0.532±0.067 | 0.941±0.228 | H4745 18 | 0.400±0.079 | 0.490±0.113 |
| H3976 | 0.638±0.079 | - | H4800_18 | 0.580±0.079 | 0.857±0.006 |
| H1790_18 | 0.461±0.033 | 1.009±0.065 | H4801_17 | 0.507±0.096 | 0.608±0.088 |
| H1791_18 | 0.409±0.034 | 0.965±0.086 | H4802_18 | 0.404±0.104 | 0.652±0.008 |
| H1994_18 | 0.358±0.061 | 0.994±0.171 | H26 | 0.331±0.101 | 0.377±0.067 |
| H1995_18 | 0.381±0.064 | 0.953±0.167 | H26_1 | 0.482±0.111 | 0.654±0.008 |
| H1996_18 | 0.422±0.080 | 0.910±0.094 | H26_2 | 0.372±0.098 | 0.641±0.036 |
| H2002_18 | 0.475±0.090 | 0.901±0.129 | H26_3 | 0.389±0.093 | 0.596±0.126 |
| H2003_18 | 0.403±0.090 | 0.908±0.074 | H26A4_1 | 0.505±0.112 | 0.479±0.152 |
| H2004_18 | 0.471±0.099 | 1.216±0.380 | H26A4_2 | 0.407±0.102 | 0.495±0.150 |
| H2005_18 | 0.505±0.092 | 1.176±0.371 | H26A4_3 | 0.365±0.130 | 0.395±0.180 |
| H2007_18 | 0.509±0.078 | 1.139±0.147 | H26A5_1 | 0.486±0.163 | 0.552±0.179 |
| H2008_18 | 0.445±0.112 | 1.139±0.306 | H26A5_2 | 0.393±0.100 | 0.595±0.112 |
| H2010_18 | 0.437±0.095 | 1.084±0.266 | H26A5_3 | 0.395±0.162 | 0.354±0.174 |
| H2576_18 | 0.405±0.099 | 0.941±0.033 | H26 cho | - | 0.161±0.110 |
| H2577_17 | 0.335±0.096 | 0.679±0.086 | H26 toco | - | 0.738±0.010 |
| H2578_18 | 0.405±0.068 | 1.216±0.276 | siRNA | 0.562±0.116 | 0.777±0.011 |
| H3974_17 | 0.563±0.065 | 1.059±0.116 | Control | 1 | 0.961±0.159 |

**[Table 9]**

| Table 9 Suppression Rate of Expression (5) | | | |
|---|---|---|---|
| ASO | Condition 5 (n=4) | Condition 7 (n=4) | Condition 9 (n=3) |
| M7 | - | - | 1.172±0.032 |
| M8 | - | - | 1.060±0.025 |
| M9 | - | - | 1.386±0.069 |
| H13 | - | - | 2.079±0.091 |
| H1687 | 0.219±0.047 | 0.371±0.083 | 1.071±0.176 |
| H1687_16_32_2 | 0.141±0.015 | 0.157±0.059 | 1.083±0.175 |
| H1687_16_32_1 | 0.227±0.010 | 0.300±0.078 | 1.105±0.175 |
| H3979_18 | 0.075±0.017 | 0.125±0.016 | 0.217±0.065 |
| H3979_18_43_1 | 0.070±0.017 | 0.091±0.046 | 0.168±0.038 |
| H3979_18_43_2 | 0.070±0.016 | 0.075±0.020 | 0.164±0.082 |
| H3979_18_43_3 | 0.061±0.014 | 0.072±0.016 | 0.213±0.066 |
| H3979_18_33_1 | 0.120±0.008 | 0.087±0.023 | 0.386±0.086 |
| H3979_18_33_2 | 0.123±0.010 | 0.107±0.020 | 0.518±0.062 |
| H4745_18 | 0.167±0.018 | 0.055±0.008 | 0.409±0.114 |
| H26 | 0.086±0.004 | 0.063±0.032 | 0.357±0.054 |
| H26A4_3 | 0.134±0.016 | 0.099±0.036 | 0.398±0.076 |
| H26A5_3 | 0.115±0.009 | 0.087±0.040 | 0.368±0.103 |
| siRNA | 0.251±0.029 | 0.078±0.026 | 0.462±0.043 |

### [Example 2] Examination of EC₅₀

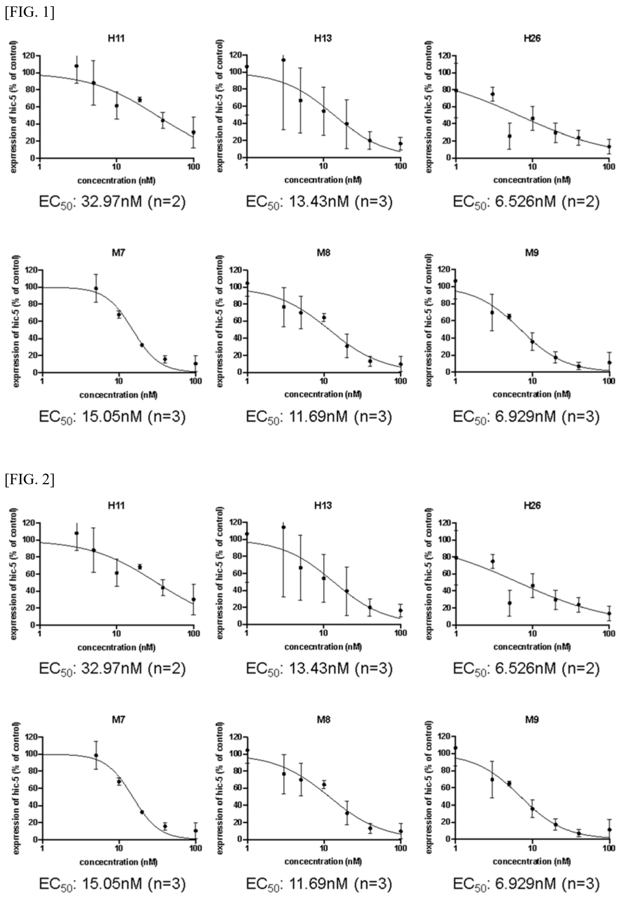

KMST-6 cells or JTC-19 cells (JCRB Cell Bank, JCRB0608) were seeded in 24-well plates at a density of 1.0×10⁴ cells/well or 2.0×10⁴ cells/well, respectively, and incubated for 24 hours. After that, ASO (1, 3, 5, 10, 20, 40, or 100 nM) was transfected using the transfection reagent (Lipofectamine^{™} RNAiMAX Transfection Reagent, Thermo Fisher Scientific). After 24 hours of incubation, the expression level of Hic-5 was quantified by RT-PCR under the same conditions as Condition 4 in Example 1, and EC₅₀ was calculated. The results are shown in FIGs. 1-2.

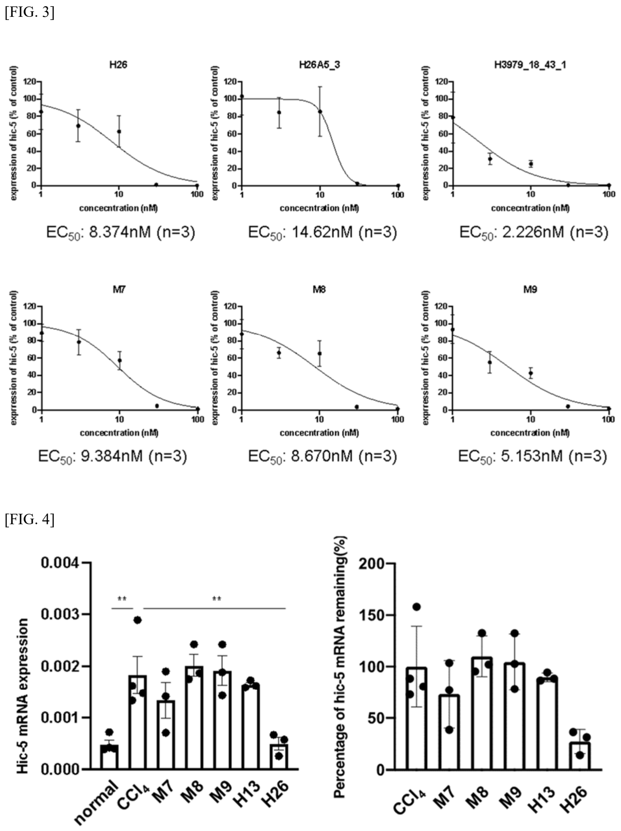

In another experiment, human HSCs were seeded in poly-L-lysine-coated 24-well plates at a density of 1.0×10⁴ cells/well and incubated for 24 hours. After that, ASO (1, 3, 10, 30, or 100 nM) was transfected using the transfection reagent (Lipofectamine^{™} RNAiMAX Transfection Reagent, Thermo Fisher Scientific). After 24 hours of incubation, the expression level of Hic-5 was quantified by RT-PCR under the same conditions as Condition 4 in Example 1, and EC₅₀ was calculated. The results are shown in FIG. 3.

### [Example 3] Toxicity Evaluation

The cytotoxicity of ASO was evaluated using ATP assay and caspase 3/7 assay.

### (1) ATP Assay

VSMC cells or NIH3T3 cells (JCRB Cell Bank, JCRM0615) were seeded in 48-well plates at a density of 0.7×10⁴ cells/well and incubated for 24 hours. After that, ASO (100 nM) was transfected using the transfection reagent (Lipofectamine^{™} RNAiMAX Transfection Reagent) and incubated for an additional 24 hours. CellTiter-Glo^{®} 2.0 Reagent (Promega) was added, and after 10 minutes of incubation at room temperature, luminescence was measured. The results are shown in Table 10 as the ratio (mean±SEM) when the control (transfection reagent only) is set to 100 (VSMC: n=8, NIH3T3: n=6).

**[Table 10]**

| Table 10 Results of ATP Assay | | |
|---|---|---|
| | VSMC | NIH3T3 |
| Untreated | 98.54±19.95 | 95.47±5.36 |
| M7 | 102.58±20.57 | 85.04±6.16 |
| M8 | 104.81±11.27 | 91.58±8.29 |
| M9 | 90.99±15.36 | 76.37±4.06 |
| H11 | 74.94±11.98 | 44.74±2.24 |
| H13 | 97.59±9.46 | 96.81±5.30 |
| H26 | 87.72±13.07 | 98.11±9.84 |

### (2) Caspase 3/7 Assay

VSMC cells or NIH3T3 cells were seeded in 96-well plates at a density of 0.6×10³ cells/well and incubated for 24 hours. After that, ASO (100 nM) was transfected using the transfection reagent (Lipofectamine^{®} 3000 Reagent, Thermo Fisher Scientific) and incubated for an additional 24 hours. Caspase-Glo^{®} 3/7 Reagent (Promega) was added, and after 30 minutes of incubation at room temperature, luminescence was measured. The results are shown in Table 11 as the ratio (mean±SEM) when the control (transfection reagent only) is set to 100 (n=2).

**[Table 11]**

| Table 11 Results of Caspase 3/7 Assay | | |
|---|---|---|
| | VSMC | NIH3T3 |
| Untreated | 98.84±10.65 | 106.93±3.59 |
| M7 | 118.72±20.58 | 96.01±4.99 |
| M8 | 120.42±10.96 | 103.55±4.77 |
| M9 | 253.67±8.86 | 123.16±16.76 |
| H11 | 457.63±16.97 | 178.88±13.11 |
| H13 | 134.54±4.60 | 99.71±3.51 |
| H26 | 124.46±23.13 | 110.02±7.37 |

### [Example 4] In Vivo Evaluation

### ASO Administration of doses

The following procedures were performed on mice (C57BL/6, 9 weeks old, male, Sankyo Labo Service).

### (i) Normal group (n=4)

On day 8, saline was subcutaneously administered to the back under isoflurane anesthesia, and on day 15, the mice were euthanized, and liver tissue and blood were collected.

### (ii) Liver fibrosis induction (CCl₄) group (n=4)

CCl₄ was intraperitoneally administered at a dose of 0.8 mL/kg on days 0, 2, 4, 7, 9, 11, and 14, and on day 8, saline was subcutaneously administered to the back under isoflurane anesthesia. On day 15, the mice were euthanized, and liver tissue and blood were collected.

### (iii) ASO treatment group (n=3)

CCl₄ was intraperitoneally administered at a dose of 0.8 mL/kg on days 0, 2, 4, 7, 9, 11, and 14, and on day 8, ASO (M7, M8, M9, H13, or H26) was subcutaneously administered to the back at a dose of 30 mg/kg under isoflurane anesthesia. On day 15, the mice were euthanized, and liver tissue and blood were collected.

### (2) Hic-5 mRNA Expression Levels

RNA was extracted from a portion of the liver tissue collected from each mouse using QuickGene-810 (Fujifilm) and QuickGene RNAtissue kit SII (Kurabo). cDNA was synthesized using ReverTra Ace^{®} qPCR RT Master Mix (Toyobo), and the expression level of Hic-5 was quantified by RT-PCR using KOD SYBR^{®} qPCR Mix (Toyobo). The results are shown in Table 12 and FIG. 4. The mRNA expression levels were analyzed using the △△CT method and are presented as values normalized to the expression level of the housekeeping gene GAPDH. The mRNA expression ratio is the ratio of the mRNA expression level of each ASO treatment group to that of the normal group set at 1, and the mRNA residual rate is the ratio of the mRNA expression level of each ASO treatment group to that of the liver fibrosis induction group set at 100. Each value represents mean±SEM.

**[Table 12]**

| Table 12 Hic-5 mRNA Expression Levels | | | |
|---|---|---|---|
| | mRNA Expression Levels | mRNA Expression Ratio | mRNA Residual Rate |
| Normal | 0.000484±0.000080 | 1.00±0.16 | - |
| CCl₄ | 0.001830±0.000359 | 3.78±0.74 | 100.00±19.63 |
| M7 | 0.001340±0.000346 | 2.77±0.71 | 73.18±18.89 |
| M8 | 0.002015±0.000209 | 4.16±0.43 | 110.06±11.42 |
| M9 | 0.001913±0.000286 | 3.95±0.59 | 104.50±15.64 |
| H13 | 0.001643±0.000043 | 3.40±0.09 | 89.75±2.36 |
| H26 | 0.000500±0.000124 | 1.03±0.26 | 27.31±6.75 |

### (3) Hic-5 Protein Expression Levels

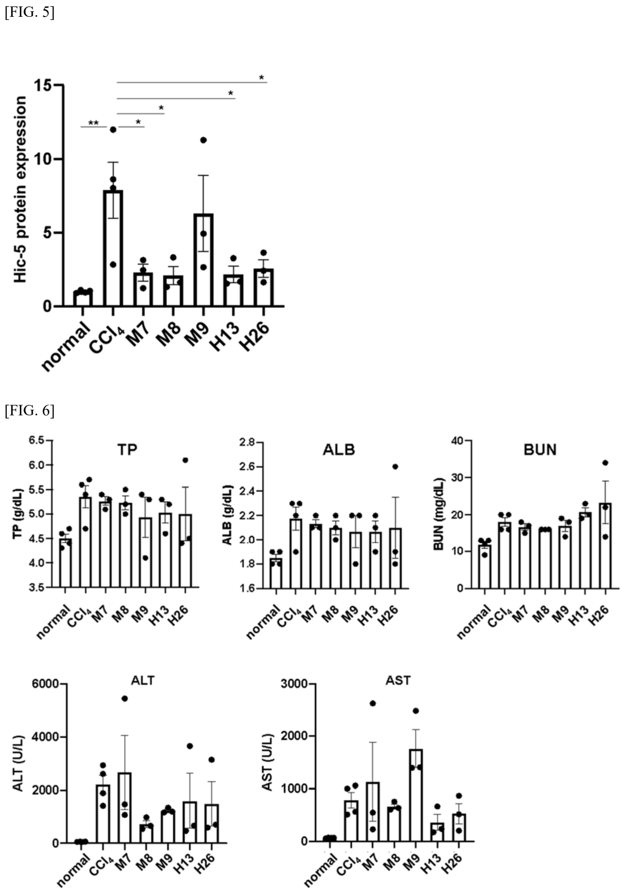

A portion of liver tissue collected from each mouse was homogenized, proteins were extracted using a tissue lysis solution containing 0.5% SDS, protein concentration was quantified using the Pierce^{™} BCA Protein Assay Kit (Thermo Fisher Scientific), and the expression levels of Hic-5 protein were quantified by Western blotting. The results are shown in Table 13 and FIG. 5. The values are based on the ratio of Hic-5 expression levels to GAPDH expression levels, with normal set as 1.

**[Table 13]**

| Table 13 Hic-5 Protein Expression Levels | |
|---|---|
| | Protein Expression Levels |
| CCl₄ | 7.87±1.89 |
| M7 | 2.28±0.56 |
| M8 | 2.09±0.62 |
| M9 | 6.29±2.58 |
| H13 | 2.17±0.56 |
| H26 | 2.57±0.59 |

### (4) Blood Biochemistry Tests

Biochemical tests were conducted on blood collected from each mouse (TP, ALB, BUN, ALT, AST). The results are shown in FIG. 6.

### (5) Quantification of Fibrotic Area

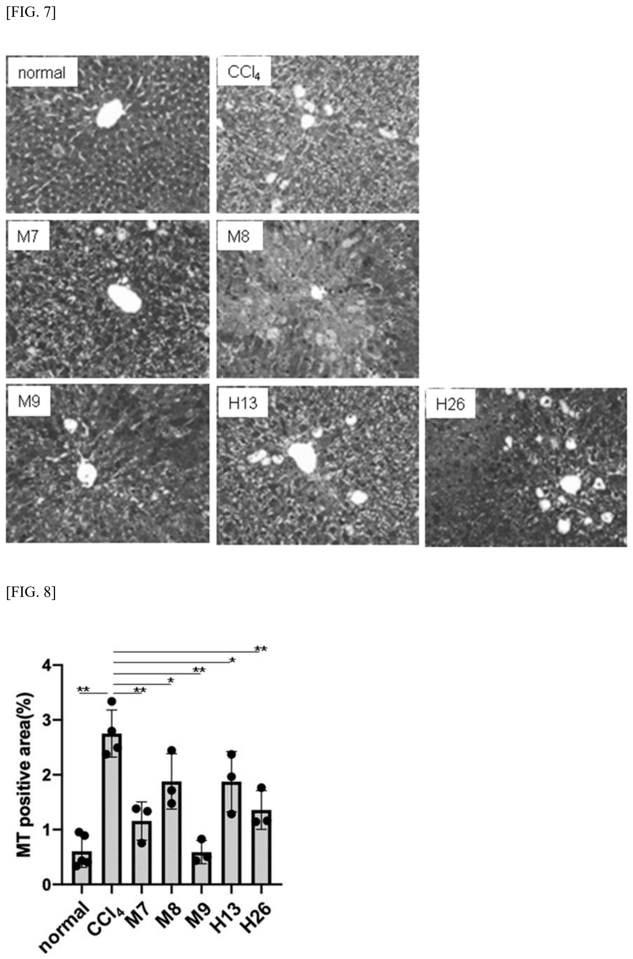

A portion of liver tissue collected from each mouse was subjected to Masson's trichrome (MT) staining, images of the perivenous area were obtained using a 20x objective lens (10 fields/mouse), and the percentage of MT-positive area (fibrotic area) in the entire field was calculated using MetaMorph^{®} imaging software (Molecular Devices). The results are shown in Table 14 and FIGs. 7-8. Statistical analysis was performed using GraphPad Prism 8.0 (GraphPad Software). Fibrosis and parenchymal damage were observed with CCl₄ administration, but a significant reduction in fibrotic area was observed with a single administration of all ASOs.

**[Table 14]**

| Table 14 Fibrotic Area | |
|---|---|
| | Fibrotic Area (%) |
| Normal | 0.605±0.132 |
| CCl₄ | 2.752±0.215 |
| M7 | 1.157±0.201 |
| M8 | 1.880±0.291 |
| M9 | 0.589±0.122 |
| H13 | 1.875±0.317 |
| H26 | 1.358±0.203 |

### [Example 5] Evaluation of On-Target Activity (2)

ASOs with the structure shown in Table 3 were designed. Synthesis was outsourced to GeneDesign Inc. The on-target activity of ASOs was evaluated under the following conditions. For H26, the one shown in Table 2 was used.

### <Condition 10>

Human HSCs (same as Example 1) were seeded at a density of 0.3×10⁴ cells/well in a 24-well plate, incubated for 24 hours, and then transfected with ASO (5 µM) using Lipofectamine^{™} RNAiMAX Transfection Reagent. After 72 hours of incubation, RNA was extracted using SuperPrep^{®} II Cell Lysis & RT Kit for qPCR2 (Toyobo), cDNA was synthesized, and the expression levels of Hic-5 were quantified by RT-PCR using KOD SYBR^{®} qPCR Mix (Toyobo) (StepOnePlus^{™}, Thermo Fisher Scientific). The results are displayed as a ratio (mean±SEM) with the control (transfection reagent only) set as 1. The primers used for RT-PCR are the same as in Example 1.

### <Condition 11>

Same as Condition 10, except VSMC cells (same as Example 1) are used.

The results are shown below.

**[Table 15]**

| | | |
|---|---|---|
| Table 15 Expression Inhibition Rate (6) | | |

| ASO | Condition 10 (n=3) | Condition 11 (n=3) |
|---|---|---|
| H26 | 0.143±0.061 | 0.474±0.077 |
| H26M1 | 0.713±0.062 | 0.576±0.089 |
| H26M2 | 0.282±0.095 | 0.474±0.082 |
| H26M3 | 0.336±0.067 | 0.548±0.103 |
| H26M4 | 0.369±0.105 | 0.445±0.097 |

### [Example 6] In Vivo Evaluation (PSC Model)

The effect of ASO on primary sclerosing cholangitis (PSC) was investigated.

### (1) Administration of ASO

The following procedures were performed on mice (C57BL/6, 9 weeks old, male, Sankyo Labo Service).

### (i) Control group (n=7)

From day 0, mice were freely fed a diet containing 0.1% DDC (3,5-diethoxycarbonyl-1,4-dihydrocollidine, Sigma-Aldrich, #137030) to create a PSC model. On days 3, 6, and 9, saline (7.5m L/kg) was subcutaneously administered to the back, and on day 10, the mice were euthanized and samples were collected.

### (ii) ASO treatment group (n=6)

From day 0, mice were freely fed a diet containing 0.1% DDC to create a PSC model. On days 3, 6, and 9, ASO (H26 from Table 2) was subcutaneously administered to the back at a dose of 3 mg/kg (7.5 mL/kg). ASO was diluted to 40 mg/mL in nuclease-free water and then further diluted 100-fold with saline for use.

### (2) Liver Weight to Body Weight Ratio

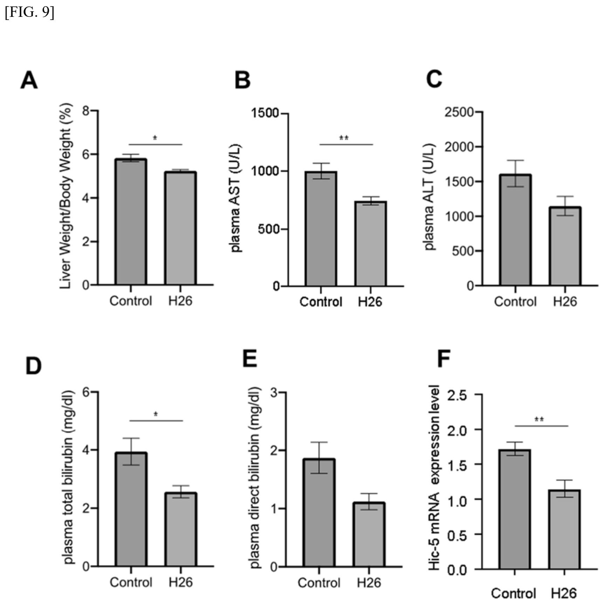

The body weight of each euthanized mouse and the weight of the liver collected as a sample were measured, and the liver weight to body weight ratio was calculated. The results are shown in FIG. 9A.

### (3) Blood Biochemistry Tests

Biochemical tests were conducted on blood collected from each euthanized mouse (plasma AST, plasma ALT, plasma total bilirubin, plasma direct bilirubin). The results are shown in FIGs. 9B-E.

### (4) Hic-5 mRNA Expression Levels

The expression levels of Hic-5 mRNA were quantified using the same method as in Example 4. The results are shown in FIG. 9F

### [Example 7] In Vivo Evaluation (MASH Model)

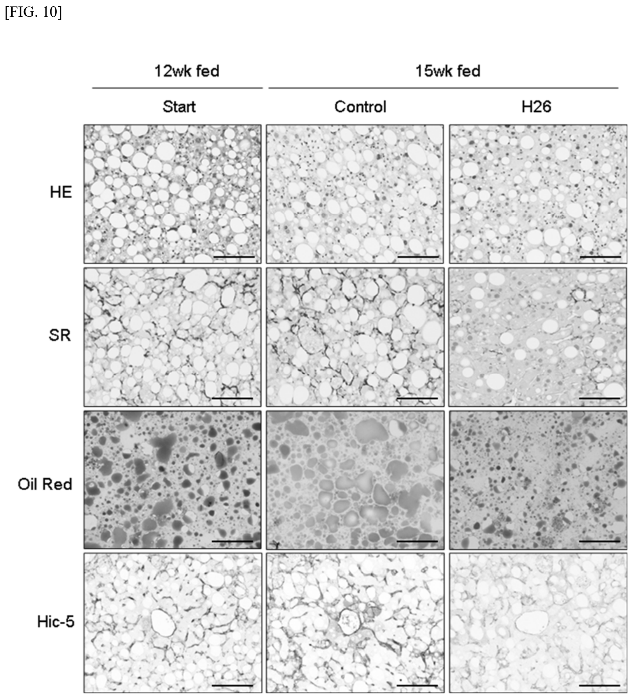

The effect of ASO on MASH was investigated. C57BL/6 mice (wild type, 11 weeks old, male) were fed a choline-deficient diet containing 60 kcal% fat and 0.1% methionine (A06071302, Research Diet Inc.). Starting 12 weeks after feeding, ASO (H26 from Table 2) was subcutaneously administered at 30 mg/body weight kg/wk for 3 weeks. ASO was diluted to 40mg/mL in nuclease-free water and then further diluted 10-fold with saline for use. The control group was administered the same amount of saline in the same manner. After the administration period, the mice were euthanized and the liver was excised. To evaluate the lesions at the start of administration, some mice were euthanized and the liver was excised 12 weeks after the start of feeding (start group). A portion of the excised liver was subjected to HE staining, Sirius Red (SR) staining, Oil Red O (ORO) staining, and Hic-5 immunostaining. For Hic-5 immunostaining, anti-Hic-5 antibody (BD Biosciences, 611165, 1:100) was used as the primary antibody, and anti-mouse IgG Alexa Fluor^{®} 647 (Abcam, ab150115, 1:500) was used as the secondary antibody. The results are shown in FIG. 10.

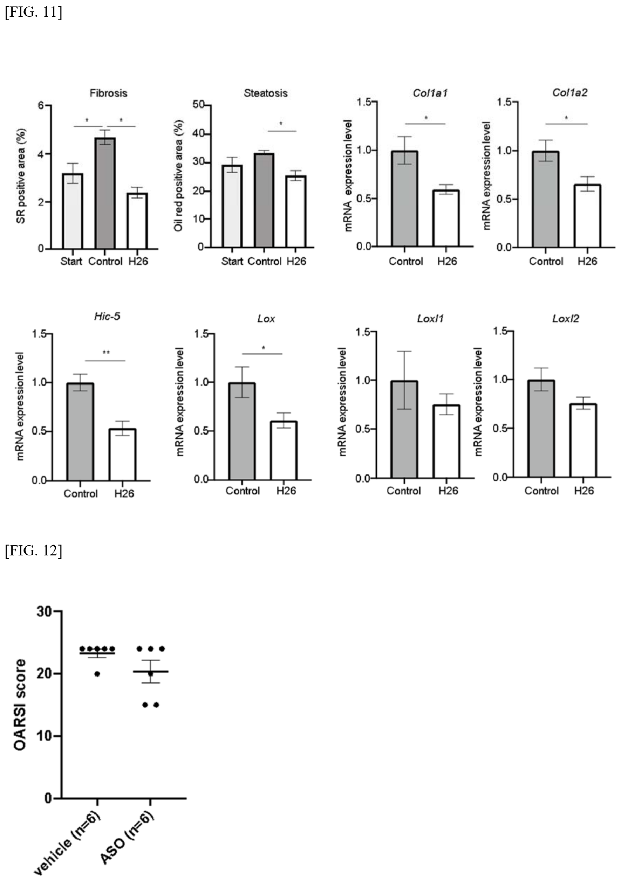

SR and ORO stained images were captured at 40x magnification using a virtual slide scanner (NanoZoomer S60, Hamamatsu Photonics), analyzed with QuPath, and the degree of fibrosis was quantified based on the area of SR-positive staining, while the degree of steatosis was quantified based on the area of ORO-positive staining. The results are shown in FIG. 11.

RNA was extracted from a portion of the collected liver tissue using QuickGene-810 (Fujifilm) and QuickGene RNAtissue kit SII (Kurabo), cDNA was synthesized using ReverTra Ace^{®} qPCR RT Master Mix (Toyobo), and the mRNA expression levels of Hic-5, Col1a1, Col1a2, Lox, Loxl1, and Loxl2 were quantified by RT-PCR using KOD SYBR^{®} qPCR Mix (Toyobo). Expression levels were analyzed using the △△CT method and displayed as values standardized based on the expression levels of the housekeeping gene GAPDH. The following primers were used. The results are shown in FIG. 11.

**[Table 16]**

| Table 16 RT-PCR Primers | | |
|---|---|---|
| Gene | | Sequence |
| *Gapdh* | S | 5'- AGGTCGGTGTGAACGGATTTG -3' (SEQ ID NO:495) |
| | AS | 5'- TGTAGACCATGTAGTTGAGGTCA -3' (SEQ ID NO:496) |
| *Hic-5* | S | 5'- ATGTCACGGTTAGGGGCTC -3' (SEQ ID NO:497) |
| | AS | 5'- GGCTTGCATACTGTGCTGTATAG -3' (SEQ ID NO:498) |
| *Col1a1* | S | 5'- AGACATGTTCAGCTTTGTGGAC -3' (SEQ ID NO:507) |
| | AS | 5'- GCAGCTGACTTCAGGGATG -3' (SEQ ID NO:508) |
| *Col1a2* | S | 5'- ACCCGATGGCAACAATGGA -3' (SEQ ID NO:509) |
| | AS | 5'- ACCAGCAGGGCCTTGTTCAC -3' (SEQ ID NO:510) |
| *Lox* | S | 5'- TCTTCTGCTGCGTGACAACC -3' (SEQ ID NO:511) |
| | AS | 5'- GAGAAACCAGCTTGGAACCAG -3'(SEQ ID NO:512) |
| *Loxll* | S | 5'- GAGTGCTATTGCGCTTCCC -3'(SEQ ID NO:513) |
| | AS | 5'- GGTTGCCGAAGTCACAGGT -3'(SEQ ID NO:514) |
| *Loxl2* | S | 5'- ATTAACCCCAACTATGAAGTGCC -3'(SEQ ID NO:515) |
| | AS | 5'- CTGTCTCCTCACTGAAGGCTC -3'(SEQ ID NO:516) |

### [Example 8] In Vivo Evaluation (OA model)

The effect of ASO on osteoarthritis (OA) was investigated. ASO administration solution was prepared by mixing H13 from Table 2 with AteloGene^{®} Local Use "Quick Gelation" (Koken, hereinafter, may be abbreviated as AteloGene^{®} QG). More specifically, 180 µL of AteloGene^{®} QG was taken into a 2 mL tube included in the kit, to which 180 µL of QG buffer included in the kit and 40 µL of ASO solution (100 µM in nuclease-free water) were added. Taking care to avoid foaming, the mixture was stirred with a rotator at about 4°C for 10 minutes at a speed of 12 rpm, and then centrifuged at about 4°C at 10,000 rpm for 1 minute. The obtained supernatant was used as the ASO administration solution and stored on ice until use. The vehicle administration solution was prepared in the same manner as the ASO administration solution, except that the ASO solution was replaced with the same amount of nuclease-free water.

Under general anesthesia, the medial collateral ligament and anterior cruciate ligament of the right hind limb of Wistar rats (7 weeks old, male, Japan SLC) were cut, and the meniscus was separated from the femur and tibia and removed (Day 0). After the procedure, each rat was maintained under the same conditions, and 40 µL of ASO administration solution or vehicle administration solution was injected into the right knee joint on days 10, 13, and 16 using a syringe (Myjector 29G, 0.5 mL syringe, Terumo Corporation). On day 19, the rats in the ASO administration group and the vehicle control group were euthanized, and the tibia was collected. The collected tissues were immersed in 10% neutral buffered formalin, fixed, embedded in paraffin, and sectioned to prepare paraffin sections. Each section was stained with Safranin-O according to standard methods, and the severity of OA was scored based on the following Grade 0-6 (depth) and Stage 0-4 (extent) (Pritzker et al., Osteoarthritis Cartilage. 2006;14(1):13-29).
Grade 0: Normal articular cartilage
Grade 1: Superficial fibrillation, or death, proliferation. Mid zone and deep zone are normal.
Grade 2: Localized fibrillation from superficial zone to mid zone, death, proliferation, increased/decreased matrix stain.
Grade 3: Fissures and matrix fibrillation extending into mid zone. Branched fissures reaching deep zone, prominent death and proliferation adjacent to fissures.
Grade 4: Loss of cartilage matrix. Erosion up to deep zone.
Grade 5: Lesion extending from calcified zone to bone. Repair by fibrocartilage.
Grade 6: Progression of bone remodeling.
Stage 0: No change
Stage 1: <10%
Stage 2: 10-25%
Stage 3: 25-50%
Stage 4: >50%

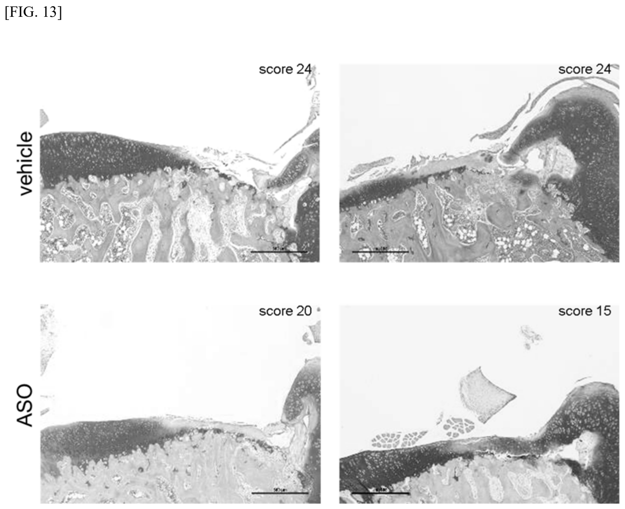

The OARSI score was calculated by multiplying the above Grade and Stage numbers, resulting in a score from 0 to 24. For example, if a site is determined to be Grade 3, Stage 2, the score is 3 × 2 = 6. The results of the OARSI score are shown in FIG. 12, and representative Safranin-O stained images of each group are shown in FIG. 13.

It is understood by those skilled in the art that numerous modifications can be made without departing from the spirit of the invention. Therefore, it should be understood that the embodiments of the invention described herein are merely illustrative and are not intended to limit the scope of the invention.

## Claims

1. An antisense oligonucleotide targeting the base sequence selected from SEQ ID NOs:157 to 312.

2. The antisense oligonucleotide according to claim 1, comprising the base sequence selected from SEQ ID NOs:1 to 156.

3. The antisense oligonucleotide according to claim 1 or 2, having modifications on the nucleotides at the 5' terminal portion and 3' terminal portion.

4. The antisense oligonucleotide according to any one of claims 1 to 3, consisting of the sequence shown in SEQ ID NOs:313 to 485.

5. A composition comprising the antisense oligonucleotide according to any one of claims 1 to 4.

6. The antisense oligonucleotide according to any one of claims 1 to 4 or the composition according to claim 5 for use in treating a disease associated with Hic-5.

7. The antisense oligonucleotide or composition for use according to claim 6, wherein the disease associated with Hic-5 is selected from vascular diseases, fibrotic diseases, cardiac hypertrophy, osteoarthritis, and tumors.

8. A composition for use in treating sclerosing cholangitis, comprising a Hic-5 inhibitor.
